# EUROPEAN PATENT SPECIFICATION

(11) **EP 4 385 491 B1**
(45) Date of publication and mention of the grant of the patent: **18.11.2026**
(21) Application number: 22213519.6
(22) Date of filing: 14.12.2022
(51) Int. Cl.: A61K 8/66, A61Q 11/00, C11D 3/386, C11D 11/00

(54) **PH-STABLE BIOTECHNOLOGY COMPOSITION BASED ON LACCASE FOR TARGETED DEGRADATION OF COLOURED MELANOIDINES AND/OR PHENOLIC ORGANIC COMPOUNDS ON DENSE SURFACES**
PH-STABILE BIOTECHNOLOGIEZUSAMMENSETZUNG AUF BASIS VON LACCASE ZUM GEZIELTEN ABBAU VON GEFÄRBTEN MELANOIDINEN UND/ODER PHENOLISCHEN ORGANISCHEN VERBINDUNGEN AUF DICHTEN OBERFLÄCHEN
COMPOSITION BIOTECHNOLOGIQUE STABLE AU PH À BASE DE LACCASE POUR LA DÉGRADATION CIBLÉE DE MÉLANOIDINES COLORÉES ET/OU DE COMPOSÉS ORGANIQUES PHÉNOLIQUES SUR DES SURFACES DENSES

(43) Date of publication of application: 19.06.2024
(73) Proprietor: SkyLab AG, 1066 Epalinges (CH)
(72) Inventor: Filatov, Viktor Andreevich, 119415 Moscow (RU); Ivanova, Angelina Dmitrievna, E161DL London (GB); Belous, Elena Yur'evna, 121309 Moscow (RU)
(74) Representative: GLAWE DELFS MOLL

(56) References cited:
- WO-A1-00/39263
- WO-A1-01/64175
- WO-A1-2020/002187
- WO-A1-97/43381
- WO-A1-97/43384
- WO-A2-2006/032724
- AU-B2- 745 711
- CN-A- 113 025 337
- US-A- 5 989 526
- US-A1- 2018 127 687
- GEORGIOU R.P. ET AL: "Decolorization of melanoidins from simulated and industrial molasses effluents by immobilized laccase", JOURNAL OF ENVIRONMENTAL CHEMICAL ENGINEERING, vol. 4, no. 1, 1 March 2016 (2016-03-01), NL, pages 1322 - 1331, XP093048667, ISSN: 2213-3437, DOI: 10.1016/j.jece.2016.01.035
- FAO: "Chemical and Technical Assessment 61st JECFA LACCASE FROM MYCELIOPHTHORA THERMOPHILA EXPRESSED IN ASPERGILLUS ORYZAE", 1 January 2004 (2004-01-01), pages 1 - 6, XP093048730, Retrieved from the Internet <URL:https://www.fao.org/fileadmin/templates/agns/pdf/jecfa/cta/61/Laccase.pdf> [retrieved on 20230523]

## Description

### TECHNICAL FIELD

The invention relates to a pH-stable biotechnology based on laccase for targeted degradation of colored melanoidins resulting from the Maillard reaction and/or phenolic food compounds, hard-to-remove biocontaminants and biostains based on them on dense surfaces. Laccase biotechnology is intended for inclusion in household products for the care of dense surfaces based on interwoven fibers of natural or artificial origin, natural materials (wood, stone), metals and various alloys with increased strength, tooth enamel based on hydroxyapatite. The use of laccase-based biotechnology makes it possible to increase the efficiency of removal fixed biocontaminants and biostains enriched with melanoidins and phenolic organic compounds, as well as to preserve the color of dense surfaces and their appearance. The composition is biodegradable, derived from fungi, particularly *Aspergillus* spp., has a safe effect on hard surfaces and on the skin, can be used to prepare household detergents for sensitive skin, such as dry, liquid, concentrated laundry detergents, surface washes, with lasting cleanliness. The use of the composition may help to reduce the surfactants caused gentle cleansing of dense surfaces while maintaining the dermatological comfort of the skin of the hands.

### THE PRIOR ART AND THE GIST THE INVENTION AS DEFINED IN THE CLAIMS

The development of household detergents with improved properties for the consumer remains one of the promising areas in the category of home care products. The global dirt and stain remover market is valued at $20.55 billion in 2019 and will grow by 4.7-5.3% annually, reaching a market size of $27.15 billion by 2025 in monetary terms [Grand View Research data, USA: https://www.grandviewresearch.com/industry-analysis/stain-remover-products-market].

Great attention to the means for removing stains on different surfaces, including fabrics, is driven by consumer hygiene and lifestyle concerns, as well as care for fabrics, in particular, their appearance and colour retention. Consumers are more likely to choose multi-surface stain removing products over standard detergents, as the first type of product offers high stain removal capacity without damaging fabric fibers and the appearance of various surfaces, while maintaining color brightness.

In recent years, ecological and environmental safety has become the main trend for such products. Consumers are paying close attention to the composition of such products and are switching from synthetic detergents to eco-friendly green household chemicals that degrade in the environment, do not accumulate in water sources, and do not damage septic tanks in country houses. In this regard, manufacturers are developing new environmentally friendly products for cleaning hard surfaces with biotechnology and biodegradable components. According to analysts, consumers from developed European countries (France, Great Britain, Germany, Italy) began to pay 39% more attention to the composition of the purchased laundry detergent and carefully study the ingredients for safety for humans and the environment [1933 people surveyed via the Kantar Profiles/Mintel; A year of innovation in fabric and dish care, 2021, Mintel]. 31% of Canadian consumers are looking for household products that are free of harmful chemical additives [1429 people surveyed via Kantar Profiles/Mintel; A year of innovation in fabric and dish care, 2021, Mintel]. 49% of consumers in Peru and other Latin American countries would like to see more ethical and effective household cleaning products with clear environmental brand initiatives at various points of sale [1000 people surveyed via Offerwise/Mintel; A year of innovation in fabric and dish care, 2021, Mintel].

An important parameter of these products remains the effectiveness, which may be less than that of synthetic products due to non-aggressive cleansing for dermatological comfort. The addition of new compositions or biotechnologies could allow achieving high degree of cleaning and stain removal on hard surfaces, color retention, while matching the trend for sustainable development of household detergents.

Some synthetic ingredients such as 1,4-dioxane or phosphates are banned and classified as carcinogens by the US Department of Health and Human Services. Similarly, ethoxylated nonylphenols are banned in the EU and Canada due to a possible estrogen-like effect on the human body, which negatively affects the human reproductive system [data from Grand View Research, USA: https://www.grandviewresearch.com/industry-analysis/stain -remover-products-market].

The most difficult stains to remove are stains of blood, sauces, grass, drinks (tea, coffee, wine) due to the presence of melanoidins and phenolic organic compounds absorbed by hard dense surfaces and oxidized into hard-to-remove intermediates. At present, the main components for removing such stains are oxygen bleaches, e.g., hydrogen peroxide, carbamide peroxide, sodium percarbonate, sodium or ammonium persulfate, which are classified as bleaches. Bleaches remove these contaminants with high efficiency but are not recommended for colored and dark fabrics due to the destruction of the dye in the fibers and a decrease in the original brightness of the products, the same is true for hard dense surfaces (tiles, glass ceramics, metal surfaces, furniture) [data from Grand View Research, USA: https://www.grandviewresearch.com/industry-analysis/stain-remover-products-market].

To achieve a high cleansing and stain-removing efficacy of products, the activity of various biotechnological compounds, in particular laccase, is being investigated. Laccase belongs to a small group of blue copper-containing oxidases that have copper in the active site of the enzyme. Copper-containing oxidases have a complex active center containing four to seven copper ions (Pooja Upadhyay et al., 2015). Laccase was first discovered in the lacquer tree Rhus variciform in 1883; however, this enzyme is widespread in higher plants, bacteria, and fungi (Yoshida, 1883; Madhavi et al., 2009; Dana et al., 2017). In the vast majority of cases laccase is industrially obtained from fungi. Among them, laccase was found in fungi from Ascomycota and Basidiomycota fyla, Deuteromycota class, and a large group of fungi that form white rot during the lignin degradation (Pooja Upadhyay et al., 2015). The activity of laccases from different fungi groups differs, so it is necessary to use laccases only from certain types of fungi.

In addition to lignin, laccase is specific to a wide range of substrates, mostly organic phenolic compounds, particularly monophenols, diphenols, methoxyphenol, aminophenols, as well as arylamines and some inorganic molecules. Oxygen acts as an electron acceptor, and water is formed as a by-product of the reaction without the formation of hydrogen peroxide and other reactive oxygen forms (Chauhan et al., 2017; Whitaker and Voragen Wong, 2003). Initially, the use of laccases was limited to phenolic compounds, but the use of mediators has significantly expanded the range of their application (Upadhyay et al., 2016). The use of mediators is due to the fact that laccase can oxidize only those substances ionization energy of which does not exceed the redox potential of the copper ion in the enzyme active center. In this case, direct oxidation of the substrate is impossible: first, laccase oxidizes the mediator, which in turn oxidizes the substrate (Morozova et al., 2007; Sahay et al., 2020; Mojtabavi et al., 2021). The ability of laccase to degrade phenolic and non-phenolic compounds has greatly expanded the area of potential application of the enzyme. It is promising in such areas as the pulp and paper and food industries, household chemicals and hygiene, medicine and cosmetology, bioremediation, as well as nanobiotechnologies and biosensors (Upadhyay et al., 2016; Dana et al., 2017).

The authors of the present invention conducted a series of studies that demonstrated the unexpected effectiveness of laccase in removing melanoidins and phenolic organic compounds on dense surfaces, particularly in the composition of household detergents. Dense surfaces are the surfaces characterized by low availability to cleaning agents due to the strong attachment of coloring compounds, and the density of which ranges from 15.07 g/m2, which corresponds to fine silk.

Melanoidins are a group of heterogeneous substances widely present in food products. Their structure is still not fully understood; however, it is known that they are a group of dark-colored high-molecular compounds that contain nitrogen in their composition. Melanoidins are formed as a result of the non-enzymatic Maillard reaction during the heat treatment of food. The Maillard reaction occurs between the carbonyl group (C=O) of carbohydrates, aldehydes and lipids and the amino group (-NH2), as well as the guanidine group (-C(NH2)2), indole and imidazole rings, amino acids. Melanoidins possess chelating properties: as a result of interaction with them, metal ions are shielded and do not enter into chemical reactions (Morales et al., 2012). The Maillard reaction accelerates with temperature increase, so coffee and bakery products account for the largest amount of melanoidins among food products (Wang et al., 2011; Kosmachevskaya, 2012; Mesías and Delgado-Andrade, 2017). They are also found in dark beer, wine, nuts and seeds, cocoa beans, soy sauce, balsamic vinegar, and other foods (Morales et al., 2012; Shaheen et al., 2021). Despite their beneficial functions-melanoidins are an important source of dietary fiber and have antioxidant properties-they can stain clothes and form stubborn stains (Eriksen et al., 1985; Iriondo-DeHond et al., 2021; Kim et al., 2022). The widespread presence of melanoidins is associated with a high incidence of such spots (Kissa, 1995; Mejia et al., 2022). Thus, the use of laccase for the degradation of melanoidins and phenolic organic compounds and the lightening of dense surfaces is of high relevance due to the frequent consumption of melanoidin-containing products by the population.

Melanoidins are found not only in food, but also in wastewater. They enter the environment from factories producing alcohols, organic acids, and food and fodder yeast and pollute it (Korni 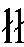 owicz-Kowalska and Rybczyńska-Tkaczyk, 2021). Such enterprises use molasses, a by-product of sugar production, as a raw material, which have a dark brown color due to the content of melanoidins in them (Georgiou et al., 2016; Zhang et al., 2019; Wei et al., 2022). Singh et al. used a wastewater model to study the effectiveness of laccase in the degradation of melanoidins and showed its low efficiency in dissolved form (Singh et al., 2015). The authors of the present invention, on the contrary, have shown the high efficiency of laccase in the degradation of melanoidins on dense surfaces. Such results may be due to differences between food and wastewater melanoidins.

Food melanoidins differ from melanoidins in wastewater and have a heterogeneous structure. Coffee melanoidins are represented by melanosaccharides, as well as methylpyrrole and furan-2-carboxyaldehyde (Morales et al, 2012; Shaheen et al., 2021). Bakery products are characterized by high content of melanoproteins (Sharma et al., 2021). Melanoidins such as fructoselysin, maltuloselysin, pyrroline, formylin, maltosine, argpyrimidine, and perlolyrine are present in dark beer (Martinez-Gomez et al., 2020). Melanoidins of nuts and seeds contain fatty acids that oxidize and interact with amino acids more efficiently than the carboxyl groups of carbohydrates. Chocolate and cocoa melanoidines are of high molecular weight, free and bound to phenolic compounds, in particular flavanols, phenolic acids, phenolic aldehyde and N-phenylpropanamide-L-amino acids (Shaheen et al., 2021). Melanoidins of tomatoes in the form of juice, puree and paste have low molecular weight due to a different phytochemical composition and technological mode of production.

It has been established that coffee melanoidins absorb electromagnetic radiation in the range of 270-450 nm (Tores de la Cruz et al., 2019), although in general melanoidins from various sources absorb in the range of 400-450 nm (Silvan M. et al., 2010). A shift to shorter wavelengths may be due to the presence of caffeine in the fraction, which is typical for coffee, cocoa, chocolate, tea, and some food products. Pure caffeine has an absorption maximum at 273 nm (Tores de la Cruz et al., 2019). Since melanoidins often contain phenolic organic compounds, in particular flavanols, phenolic acids, and aldehydes (Shaheen et al., 2021), it is necessary to additionally degrade phenolic organic compounds. The most frequent component of high molecular weight melanoidins is chlorogenic acid, which has an absorption maximum at 326 nm (Tores de la Cruz et al., 2019), and active phenolic acids, flavones and their glycosides with absorption maxima of 288, 296, 302, 322, 325, 337 nm (Masek A. et al., 2020).

The inventors discovered the innovative effectiveness of laccase in the degradation of melanoidins and phenolic organic compounds on dense surfaces. It is of practical importance and of particular interest due to the relevance of this problem. Laccase effectively breaks down food melanoidins present in many foods that humans contact on a daily basis, on dense surfaces such as clothing and textiles and teeth enamel.

One of the limitations to the use of laccase in household detergents for cleaning dense surfaces is a biochemical factor, particularly, the co-presence with proteases that cleave peptide bonds in protein contaminants (blood, grass, eggs, milk) and can cleave bonds in the structure of the laccase enzyme. One of the most common examples of proteases in household detergents is subtilisin. Subtilisin is a proteolytic enzyme from the class of serine proteinases. It is produced by bacteria predominantly of the Bacillus genus, in particular Bacillus subtilis, to protect against other microorganisms. Subtilisin is involved in the breakdown of protein via peptide bonds hydrolysis. It initiates a nucleophilic attack on the peptide bond via a serine residue located in its active site (Marget et al., 2022). Subtilisin is widely used in household detergents, cosmetics, food processing, and pharmaceuticals (Veluchamy et al., 2011).

Since subtilisin is a protease that cleaves proteins, including laccases, it is used in stability experiments. Cruz-Vázquez A et al. investigated the stability and activity of laccase in the presence of protease. The authors showed that laccase activity decreases in the presence of protease. The use of protease inhibitors makes it possible to increase the activity of laccase, which indicates a direct inhibition of laccase activity by the protease (Cruz-Vázquez A et al., 2022). Similarly, the presence of chelating agents, in particular EDTA, has been found to inhibit laccase activity (Cruz-Vázquez A et al., 2022).

Despite the conflict between protease (subtilisin) and laccase described in the literature, the inventors unexpectedly found compatibility between subtilisin and biolaccase obtained from fungi of the Aspergillus spp. genus. With the combined use of both components, a high cleaning and stain-removing ability was observed on different dense surfaces.

An additional limitation to the use of laccase in household chemicals for cleaning dense surfaces is pH sensitivity, namely the enzymatic activity of laccase at various pH values. Most fungal laccases are active in the pH range of 3.0-5.5 and become inactive in the neutral or alkaline pH range, although the structure is stable even at pH 7.0 (Xu F., 1997; Baldrian P., 2006). The most favorable pH range for the currently known laccases is 4.8-5.0 (Vats A., 2022; Kumar et al., 2016). Unfortunately, scientists have failed to create a stable laccase active at neutral and alkaline pH (Maté et al. 2010, 2013a, b; Mate and Alcalde 2015; Novoa et al. 2019).

Despite the described conflict between the pH range and laccase the inventors unexpectedly found a high activity of biolaccase obtained from fungi of the Aspergillus spp. genus in cleavage of melanoidins in the pH range of 3.0-8.0. When using laccase at pH 7.0-8.0, a high cleaning and stain removal ability was observed on dense surfaces in the composition of household detergents.

The technical result of biotechnology based on laccase is the effective removal of biostains enriched with melanoidins and phenolic organic compounds, maintaining the brightness of the color of various fabrics and maintaining the enzymatic stability of laccase in the presence of protease (subtilisin) in cleaning products for dense surfaces. The biotechnology is active in the pH range, in particular 3.0-8.0 units, and in a wide temperature range, in particular from +10 to +60°C, which expands the scope of application in eco-friendly household detergents in order to conserve the planet's resources and ensure sustainable development of the industry. Laccase allows targeted degradation of melanoidins and phenolic organic compounds in biostains and contaminants on dense surfaces when used in various household detergents or oral care products. Additionally, color retention is ensured. In this case, laccase is understood as a raw material containing the corresponding pure active components, as well as technical impurities that could be formed during the production of the target raw material. Thus, the use of biolaccase in household detergents leads to an increase in the kinetics of the degradation of complex biological contaminants and stains based on melanoidins and phenolic organic compounds by increasing the activity of laccase in the required pH range of 3.0-8.0, which ensures rapid stain removal from dense surfaces even in cold water conditions. A distinctive feature is that the component in the claimed concentrations acts only against complex organic contaminants and does not impair the consumer appearance of most fabrics, in particular cotton, synthetic, mixed, delicate. Laccase is compatible with various components in household chemicals, in particular anionic, amphoteric, nonionic surfactants, preservatives, solvents, bioenzymes, extracts, aromatic components, thus it is possible to use detergents and surface cleaners with this biotechnology on a regular basis with stability of laccase during storage. The use of laccase ensures complete care for various dense surfaces in one household chemical product or oral care products for daily use.

Thus, the invention as a whole relates to laccase and its use, which makes it possible to achieve such technical results as the effective enzymatic degradation of biostains based on melanoidins and phenolic organic compounds while maintaining the color of dense surfaces in the ΔL*a*b coordinates, which are not achieved or insufficiently achieved by modern commercially available means in the art.

WO 97/43381 A1 and WO 97/43384 A1 disclose liquid compositions having a pH of 7 or 7.5, whereby said compositions comprise a laccase and a protease.

WO 2020/002187 A1 relates to detergent compositions comprising a laccase and optionally a protease. The exemplified composition is liquid. The detergent composition can have pH of 7.5. The laccase acts on phenolic stains such as anthocyanins.

WO 2006/032724 A2 discloses a laccase which functions at pH 3 to 9 and can be used to remove stains from e.g. fabrics, such as grass soiling or tea soiling.

US 2018/127687 A1 discloses the use of laccase in a detergent composition for addressing anthocyanin-containing stains. Optionally a protease enzyme can be present.

WO 00/39263 A1 discloses the use of a laccase to remove tea and coffee stains 'from cotton fabrics at a pH of 7.95.

AU 745 711 B2 discloses the use of a laccase to remove tea and coffee stains from cotton fabrics at a pH of 4.5.

US 5 989 526 A discloses the use of a laccase to clean tea or coffee stains from teeth. The laccase is applied from a composition having a pH from 5-9, which can further optionally comprise a protease.

WO 01/64175 A1 discloses the use of a laccase to clean tea or coffee stains from teeth. The laccase is applied from a composition having a pH of 4.

Georgiou et al., Decolorization of melanoidins from simulated and industrial molasses effluents by immobilized laccase, Journal of Environmental Chemical Engineering 4 (2016) 1322-1331 describes the decolorization of melanoidins from simulated and industrial molasses effluents by immobilized laccase and refers to the use of "free" laccase. The test is done at a pH within the range of 3-8.

CN 113 025 337 A discloses an aqueous solution of pH 4.5 comprising a laccase produced from *Aspergillus oryzae,* for use in a method of soil remediation.

### ESSENCE OF INVENTION

The melanoidins can color different types of dense surfaces, in particular fabrics and tooth enamel based on hydroxyapatite. Laccase bonds with wool and polyamide through electrostatic interaction with amino groups on their surface and with cotton through hydrogen bonding (Mongkholrattanasit et al., 2021). In this regard, the specific degradation of coloured melanoidins in textiles and other dense surfaces is of particular interest. As the authors found out, laccase has such specificity and effectiveness against coloured melanoidins and, additionally, phenolic organic compounds. However, the use of laccase in household detergents has been presented in several patents, highlighted below.

Patent application WO2020002187 [HENKEL AG & CO KGAA (DE)], published on January 02, 2020, claims laccase from a strain of basidiomycetes *Marasmiellus palmivorus,* as well as its variants. In particular, the use of the original laccase as well as laccases that match its original amino acid sequence by at least 70% is described. The patent application notes that laccase variants can be obtained as a result of modifications of the parent laccase by one or more amino acid substitutions, fragmentation, deletion, insertion or substitution and coincide with the parent molecule by a certain number of contiguous amino acids. Also claimed is the use of laccase in the composition of household detergents and surface washes for textiles and other hard surfaces as tooth enamel with high density, against stains containing anthocyanins, but not melanoidins. In tests to assess the activity of laccase against stains, blueberry juice (CFT, CS-15) and wild berry ice cream (CFT, CS-72) were used as a contaminant, where anthocyanins are the predominant pigments and melanoidins are fully absent (Kallam et al., 2017).

In the patent family, which includes international patents WO9743381, WO9743382 and WO9743383 [PROCTER & GAMBLE (US); HERBOTS IVAN MAURICE ALFONS JA [BE]; BARNABAS MARY VIJAYARANI (US); BUSCH ALFRED (BE); DENOODT KRISTEL (BE)], claims the use of laccase in various formulations. Among them, the joint use of laccase with: cellulase, a polymer that inhibits the transfer of dyes, and other components common among the above patents. Applications of the claimed composition include cleaning, softening, maintaining the whiteness and color of fabrics, removing stains, inhibiting the transfer of dyes, treating and cleaning hard surfaces, and using it as a dishwashing detergent. However, the Applicant did not specify the type of stains against which the composition is used, and cited possible compositions of the components as examples, but did not detail the use as claimed agents.

The inventors have also found several features of the laccase found in the hard surface wahes that support its effectiveness. The activity of laccase in free form belongs to pH of 3.0-5.0. The pH optimum depends on the substrate, but in the case of laccases from most fungal strains, it belongs to the range at pH of 4.0-5.0 that laccase reaches its maximum activity (Desai et al., 2011; Mazlan and Hanifah, 2017; Kolomytseva et al., 2017). Nevertheless, the authors found that the selected laccase from the *Aspergillus spp.* in the composition of the agent for cleaning dense surfaces, it retains its activity at pH of 3.0-7.0, and the working range of the enzyme belongs to the region of pH 3.0-8.0. Thus, the selected laccase retains its activity in a wider pH range, which expands the scope of its application.

The inventors have also found that laccase activity is not reduced by the addition of a protease, in particular subtilisin, to detergents. Subtilisin belongs to a group of serine proteases that cleave proteins by hydrolysis of peptide bonds. In the presence of protease inhibitors, an increase in laccase activity was shown, which indicates a negative effect of proteases on laccase activity (Cruz-Vázquez et al., 2022). However, the Inventors did not find such an effect when subtilisin was added to the detergents, indicating that this protease does not reduce the activity of the selected laccase and retains their activity when present together. Thus, the use of laccase, stable in the pH range of 3.0-8.0, for the degradation of coloured melanoidins and/or phenolic organic compounds in the field of household detergents has not been presented before and it is innovative approach in technical field of household and oral care. Effective enzymatic cleavage of stains based on coloured melanoidins and/or phenolic organic compounds is not achieved or not sufficiently achieved by modern commercially available means in the present technical field.

In a first aspect, the invention relates to a biolaccase intended for use in household detergents and oral care products for the cleavage of melanoidins and/or phenolic organic compounds on dense surfaces, where the specified biolaccase has a biological activity greater than 1000 U/g and is active at pH of 3.0-8.0 at 25 °C, and a temperature of 10-60°C.

In a second aspect, the invention relates to the use of the laccase according to the invention in a household detergent for laundry washing. The household detergent according to the invention may contain 0.03 to 1.00 wt.% of the composition according to the invention.

Said laccase comprising ccomposition is in particular intended for use in laundry detergents and is active at pH of 3.0 to 7.0 and at a water hardness of 0-15.0 °dH, corresponding to "soft" and "medium" hardness (while higher water hardness is not excluded, even though less desired).

In third aspect, the invention relates to the use of the laccase according to the invention in an oral care products, as toothpastes, mouthwashes, and oral foams, for teeth cleaning and whitening. The oral care products according to the invention may contain 0.03 to 1.00 wt.% of the composition according to the invention.

In one embodiment of an invention, the laccase may be a biotechnologically derived laccase from fungi.

In another embodiment of an invention, the laccase may be biotechnologically derived laccase from fungi, in particular Aspergillus spp.

The composition may differ in that said laccase is in a water-glycerol or water-sorbitol or water-glycerol-sorbitol or glycerol-sorbitol or water-propylene glycol or water-maltitol solution, or in a powdered or granulated form with various excipients.

In another aspect, the invention relates to a laundry detergent containing 0.03-1.00 wt.% of the composition according to the invention.

The invention may be characterized in that said household detergent is selected from laundry detergent, including delicate laundry detergent or baby and child laundry detergent, laundry conditioner, stain remover for pre-spotting and washing laundry, laundry gel and laundry conditioner.

The laundry detergent may be characterized in that the detergent in question is selected from a delicate laundry detergent and a baby or child laundry detergent.

The laundry detergent may be characterized in that the detergent in question is selected from a detergent for membrane fabrics and a detergent for sportswear.

The laundry detergent may be characterized in that the detergent in question is a powder laundry detergent.

In another aspect, the invention relates to a surface wash containing 0.03-1.00 wt.% of the composition according to the invention.

In another aspect, the invention relates to a dishwashing liquid containing 0.03-1.00 wt.% of the composition according to the invention.

In another aspect, the invention relates to a dishwasher tablet containing 0.03-1.00 wt.% of the composition according to the invention.

In another aspect, the invention relates to an oral care product containing 0.03-1.00 wt.% of the composition according to the invention.

The oral care products may be characterized in that the product is selected from a toothpaste and a mouthwash and a foam.

In another aspect, the invention relates to the use of a composition according to the invention for enzymatic degradation of coloured melanoidins and/or phenolic organic compounds, biostains and soils on them on various dense surfaces.

The use may be characterized in that said dense surface is selected from cotton, synthetic, blended, delicate (wool, silk, cashmere, merino, down, feather and their mixtures), membrane, teeth enamel based on hydroxyapatite.

The invention will be further explained in detail by way of specific embodiments and illustrated by exemplary realisations.

### DETAILED DESCRIPTION OF THE INVENTION

In a first aspect, the invention relates to a biolaccase intended for use in household detergents and oral care products for the cleavage of melanoidins and/or phenolic organic compounds on dense surfaces, where the specified biolaccase has a biological activity greater than 1000 U/g and is active at pH of 3.0-8.0 at 25 °C, and a temperature of 10-60°C.

The mass content of laccase in said household detergents may be 0.03, 0.05, 0.1, 0.2, 0.3, 0.4, 0.5, 0.6, 0.7, 0.8, 0.9 or 1.0 or any value in between.

The mass content of laccase in said oral care products may be 0.03, 0.05, 0.1, 0.2, 0.3, 0.4, 0.5, 0.6, 0.7, 0.8, 0.9 or 1.0 or any value in between.

The invention may be characterized in that the laccase is a laccase obtained biotechnologically from microorganisms, in particular fungi or yeasts. The laccase can be a substance or a commercially available product with the registration number CAS 80498-15-3.

The present invention also relates to the use of the composition according to the invention in a household detergent.

The household detergent according to the invention may contain 0.03-1.00 wt.% of the composition according to the invention. For example, the household detergent according to the invention may contain 0.03, 0.05, 0.1, 0.2, 0.3, 0.4, 0.5, 0.6, 0.7, 0.8, 0.9 or 1.0 wt.% or any value in between of said compositions according to the invention.

The present invention also relates to a laundry agent, a laundry agent for sportswear or a laundry agent for baby's or children's laundry containing 0.03-1.00 wt.% of the composition according to the invention.

The present invention also relates to a powder detergent for washing white and coloured laundry containing 0.03-1.00 wt.% of the composition according to the invention.

In the composition according to the invention, the acceptable excipients may be selected from the following categories of components.

The present invention also relates to the use of the composition according to the invention in an oral care product.

The present invention also relates to the use of the laccase of the invention for the cleavage of coloured melanoidins and/or phenolic organic compounds, biocontaminants and biostains based on them on various dense surfaces, including cotton, synthetic, blended, membrane, tooth enamel, to maintain long-term cleanliness and whiteness.

The composition preferably does not contain other active agents and/or excipients, such as washing active agents and/or acceptable excipients but may contain them. Such agents may represent or represent agents traditionally used in the art and which are known to the skilled person in the art. The addition of said agents to the composition of the complex according to the invention does not invalidate the achievement of the claimed technical results but can improve them.

Within the scope of the invention are also household detergents such as laundry detergent, laundry detergent for sportswear, laundry detergent for baby's or children's laundry, powdered laundry detergent, dishwashing liquid, surface cleaner, dishwasher tablet and oral are products such as toothpaste, mouthwash, or foam.

All mass fractions, mass parts, mass percentages as well as volume fractions, volume parts, volume percentages in the present disclosure are given in relation to the preparation, agent, composition or product to which they relate in the context specified.

In a household detergent according to the invention, the acceptable excipients may be selected from the following categories of components.

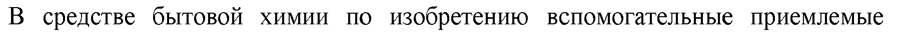 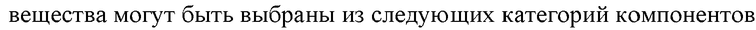 .

### Anionic surfactants:

Salts of higher carboxylic acids with the general formula: R1-CO2X1, where R1 is an alkyl and / or alkenyl group with a long hydrocarbon chain from 5 to 21 atoms unexpectedly, and X1 is an alkali and/or alkaline earth metal, ammonium, alkylammonium, alkanolammonium, glucoammonium cation, basic amino acids;
Alkyl polyethylene glycol sulfate with the general formula R2-O(-CH2-CH2-O)n1 (SO3) n2 X1, wherein n1 takes a value from 0 to 10 and denotes the number of polyethylene groups, R2 is an alkyl and/or alkenyl group with a hydrocarbon chain length of 6 to 22 carbon atoms, X1 is an alkali and/or alkaline earth metal cation, ammonium, alkylammonium, alkanolammonium, glucoammonium, basic amino acid;
Alkyl sulfate with the general formula R3-OSO3X3, where R3 is an alkyl and / or alkenyl group with a long hydrocarbon chain from 6 to 22 carbon atoms, and X3 is an alkali and/or alkaline earth metal, ammonium, alkylammonium, alkanolammonium, glucoammonium cation;
Amide salt of a higher fatty acid and methylglycine with the general formula R4-C(O)-N(-CH3)-CH2-CO2X4, wherein R4 is an alkyl and/or alkenyl group with a hydrocarbon chain length from 5 to 21 carbon atoms and X4 is an alkali and/or alkaline earth metal cation, ammonium, alkanolammonium, glucoammonium;
An alkyl polyethylene glycol carboxylate with the general formula: R5-O(-CH2-CH2-O-)n2CH2-CO2X5, wherein n2 can take values from 1 to 15 and denotes the number of polyethylene glycol groups, R5 is an alkyl and/or alkenyl group with a hydrocarbon chain length of 6 to 22 carbon atoms and X5 is an alkali and/or alkaline earth metal cation, ammonium, alkylammonium, alkanolammonium, glucoammonium;
A bisubstituted salt of 2-sulfo carboxylic acid with the general formula: R6-CH(-SO3X6)-CO2X6, wherein R6 is an alkyl and/or alkenyl group with a hydrocarbon chain length of 4 to 20 carbon atoms and X6 is an alkali and/or alkaline earth metal cation, ammonium, alkylammonium, alkanolammonium, glucoammonium;
A mono- or divalent amide salt of a higher carboxylic acid and glutamic acid with the general formula: R7-C(O)-NH-CH(-CH2-CH2-CO2X7)-CO2X7, wherein R7 is an alkyl and/or alkenyl group with a hydrocarbon chain length of 5 to 21 carbon atoms and X7 is an alkali and/or alkaline earth metal cation, ammonium, alkylammonium, alkanoammonium, glucoammonium or hydrogen;
An amide salt of a higher fatty acid and glycine with the general formula: R8-C(O)-NH-CH2-CO2X8, wherein R8 is an alkyl and/or alkenyl group with a hydrocarbon chain length of 5 to 21 carbon atoms, and X8 is an alkali and/or alkaline earth metal cation, ammonium, alkylammonium, alkanolammonium, glucoammonium;
An amide salt of a higher fatty acid and alanine with the general formula: R9-C(O)-NH-CH(-CH3)-CO2X9, wherein R9 is an alkyl and/or alkenyl group with a hydrocarbon chain length of 5 to 21 carbon atoms and X9 is an alkali and/or alkaline earth metal cation, ammonium, alkylammonium, alkanolammonium, glucoammonium;
An amide salt of a higher fatty acid and 2-aminomethylethanesulfonic acid with the general formula: R10-C(O)-N(-CH3)-CH2-CH2-SO3X10, wherein R10 is an alkyl and/or alkenyl group with a hydrocarbon chain length of 5 to 21 carbon atoms, and X10 is an alkali and/or alkaline earth metal cation, ammonium, alkylammonium, alkanolammonium, glucoammonium;
An alkylpolyglucoside hydroxypropyl sulfonate with the general formula: R11-O-[G]p1-O-CH2-CH(-OH)-CH2-SO3X11, wherein R11 is an alkyl and/or alkenyl group with a hydrocarbon chain length of 6 to 22 carbon atoms, G is a saccharide fragment containing 5 or 6 carbon atoms, p1 can take values from 1 to 4, and X11 is an alkali and/or alkaline earth metal cation, ammonium, alkylammonium, alkanolammonium, glucoammonium;
An alkylpolyglucoside carboxylate with the general formula: R12-O-[G]p2-O-CH2-CO2X12, wherein R12 is an alkyl and/or alkenyl group with a hydrocarbon chain length of 6 to 22 carbon atoms, G is a saccharide fragment containing 5 or 6 carbon atoms, p2 can take values from 1 to 4, and X12 is an alkali and/or alkaline earth metal cation, ammonium, alkylammonium, alkanolammonium, glucoammonium;
An amide salt of a higher fatty acid and a threonine with the general formula: R13-C(O)-NH-CH(-CH(-OH)-CH3)-CO2X13, wherein R13 is an alkyl and/or alkenyl group with a hydrocarbon chain length of 5 to 21 carbon atoms, and X13 is an alkali and/or alkaline earth metal cation, ammonium, alkylammonium, alkanolammonium, glucoammonium;
An amide salt of a higher fatty acid and an amino acid obtained by hydrolysis of proteins from vegetable raw materials, with the general formula: R14-C(O)-AAX14, wherein R14 is an alkyl and/or alkenyl group with a hydrocarbon chain length of 5 to 21 carbon atoms, AA is an amino acid or peptide obtained by hydrolysis of plant protein (possible protein sources: apple, soybean, wheat, cotton etc.), and X14 is an alkali and/or alkaline earth metal cation, ammonium, alkylammonium, alkanolammonium, glucoammonium.

### Amphoteric surfactants:

A divalent salt of acylamphodiacetate with the general formula: R15-C(O)-NH-CH2-CH2-N(-CH2-CO2X15)-CH2-CH2-O-CH2-CO2X15, wherein R15 is an alkyl and/or alkenyl group with a hydrocarbon chain length of 5 to 21 carbon atoms and X15 is an alkali and/or alkaline earth metal cation, ammonium, alkylammonium, alkanolammonium, glucoammonium;
An acylamphoacetate salt with the general formula: R16-C(O)-NH-CH2-CH2-N(-CH2-CO2X16)-CH2-CH2-OH, wherein R16 is an alkyl and/or alkenyl group with a hydrocarbon chain length of 5 to 21 carbon atoms and X16 is an alkali and/or alkaline earth metal cation, ammonium, alkylammonium, alkanolammonium, glucoammonium;
An alkylamphoacetate salt with the general formula: R17-C(=N-CH2-CH2-N((-CH2-CH2-OH)-CH2-CO2X17)-), wherein R17 is an alkyl and/or alkenyl group with a hydrocarbon chain length of 5 to 21 carbon atoms and X17 is an alkali and/or alkaline earth metal cation, ammonium, alkylammonium, alkanolammonium, glucoammonium;
An acylamidoalkylbetaine with the general formula: R18-C(O)-NH-R19-N(-CH3)2)-CH2-CO2, wherein R18 is an alkyl and/or alkenyl group with a hydrocarbon chain length of 5 to 21 carbon atoms, R19 is an alkyl group with a hydrocarbon chain length of 1 to 4 carbon atoms;
An acylamidoalkylhydroxysultaine with the general formula: R20-C(O)-NH-R21-N(-CH3)2-CH2-CH(-OH)-CH2-SO3, wherein R20 is an alkyl and/or alkenyl group with a hydrocarbon chain length of 5 to 21 carbon atoms, R21 is an alkyl group with a hydrocarbon chain length of 1 to 4 carbon atoms;
An acylamidoalkylamine oxide with the general formula: R22-C(O)-NH-R23-N(-CH3)2-O, wherein R22 is an alkyl and/or alkenyl group with a hydrocarbon chain length of 5 to 21 carbon atoms, R23 is an alkyl group with a hydrocarbon chain length of 1 to 4 carbon atoms;
An alkylbetaine with the general formula: R24-N(-CH3)2)-CH2-CO2, wherein R24 is an alkyl and/or alkenyl group with a hydrocarbon chain length of 5 to 21 carbon atoms;
An alkylhydroxysultaine with the general formula: R25-N(-CH3)2-CH2-CH(-OH)-CH2-SO3, wherein R25 is an alkyl and/or alkenyl group with a hydrocarbon chain length of 6 to 22 carbon atoms;
An alkylsultaine with the general formula: R26-N(-CH3)2-CH2-CH2-CH2-SO3, wherein R26 is an alkyl and/or alkenyl group with a hydrocarbon chain length of 6 to 22 carbon atoms;
An alkylamine oxide with the general formula: R27-N(-CH3)2-O, wherein R26 is an alkyl and/or alkenyl group with a hydrocarbon chain length of 6 to 22 carbon atoms.

### Nonionic surfactants:

An alkyl glucoside with the general formula: R28-O-[G]p3, wherein R28 is an alkyl and/or alkenyl group with a hydrocarbon chain length of 4 to 22 carbon atoms, G is a saccharide fragment containing 5 or 6 carbon atoms, p3 can take values from 1 to 4;
An alkylpolyethylene glycol with the general formula: R29-O(-CH2-CH2-O-)n3H, wherein n3 can take values from 2 to 20, and denotes the number of polyethylene glycol groups, R29 being an alkyl and/or alkenyl group with a hydrocarbon chain length of 6 to 22 carbon atoms;
An alkylpolyethylene/propylene glycol with the general formula: R30-O(-CH2-CH2-O-)n4(-CH(-CH3)-CH2-O-)n5H, wherein n4 can take values from 2 to 20 and denotes the number of polyethylene glycol groups, n5 can take values from 2 to 20 and denotes the number of polypropylene glycol groups, R30 is an alkyl and/or alkenyl group with a hydrocarbon chain length of 6 to 22 carbon atoms;
A dialkyl polyethylene glycol with the general formula: R31-O(-CH2-CH2-O-)n6R32, wherein n6 can take values from 2 to 20, and denotes the number of polyethylene glycol groups, R31 being an alkyl and/or alkenyl group with a hydrocarbon chain length of 6 to 22 carbon atoms, R32 being an alkyl and/or alkenyl group with a hydrocarbon chain length of 1 to 12 carbon atoms;
A dialkylpolyethylene/propylene glycol with the general formula: R33-O(-CH2-CH2-O-)n7(-CH(-CH3)-CH2-O-)n8-R34, wherein n7 can take values from 2 to 20, and denotes the number of polyethylene glycol groups, n8 can take values from 2 to 20, and denotes the number of polypropylene glycol groups, R33 is an alkyl and/or alkenyl group with a hydrocarbon chain length from 6 to 22 carbon atoms, R34 is an alkyl and/or alkenyl group with a hydrocarbon chain length from 1 to 12 carbon atoms.

### Dispersing medium for the polysaccharide/solvent:

An organic alcohol with the general formula: R35(-OH)s1, wherein R35 is an alkyl group with a hydrocarbon chain length from 3 to 12 carbon atoms, s1 can take values from 1 to 12, and denotes the number of hydroxyl groups arranged randomly in a hydrocarbon radical relative to each other;
An alkylpolypropylene glycol with the general formula: H(-CH(-CH3)-CH2-O-)n9R36, wherein n9 can take values from 2 to 10 and denotes the number of polypropylene glycol groups, R36 is an alkyl group with a hydrocarbon chain length from 1 to 10 carbon atoms.

### pH regulators:

Organic acids with the general formula: R37(-OH)s2(-COOH)m1, wherein R37 is an alkyl group with a hydrocarbon chain length of 1 to 12 carbon atoms, s2 can take values from 1 to 12 and denotes the number of hydroxyl groups arranged in the hydrocarbon radical in random order with respect to each other, m1 can take values from 1 to 4 and denotes the number of carboxyl groups arranged in the hydrocarbon radical in random order with respect to each other;
Solutions of alkali or alkaline earth metal hydroxides, ammonia, primary and tertiary alkylamines, primary and tertiary alkanolamines, primary and tertiary glucamines, basic amino acids, disodium salt of citric acid, trisodium salt of citric acid.

### Inhibitors of the reverse sedimentation of contamination:

Polysaccharide derivatives: sodium salt of carboxymethyl polysaccharide, hydroxyalkyl polysaccharide, alkyl polysaccharide and cellulose gum;
Polyvinylpyrrolidone and its derivatives, copolymers of polyvinylpyrrolidone and vinylimidazole;
Water soluble salts of polyacrylic acid, polymethacrylic acid, copolymers of acrylic/methacrylic and maleic acid.

### Defoamers:

Higher carboxylic acids with the general formula: R39-CO2H, wherein R39 is an alkyl and/or alkenyl group with a hydrocarbon chain length of 5 to 21 carbon atoms;
Higher carboxylic alcohols with the general formula: R40-COH, wherein R40 is an alkyl and/or alkenyl group with a hydrocarbon chain length of 5 to 21 carbon atoms;
Esters of higher carboxylic alcohols with the general formula: R41-O-R42, wherein R41, R42 is an alkyl and/or alkenyl group with a hydrocarbon chain length of 4 to 22 carbon atoms;
Bisamides of alkyl diamines and higher carboxylic acids with the general formula: R43-C(O)-NH-R44-NH-C(O)-R45, wherein R43, R45 is an alkyl and/or alkenyl group with a hydrocarbon chain length of 5 to 21 carbon atoms and R44 an alkyl radical with a hydrocarbon chain length of 1 to 12 carbon atoms;
Builders and fillers: sodium silicate, sodium sulfate, sodium chloride, sodium carbonate and other commercially available components.

### Preservatives:

Organic acids and salts of alkali and alkaline earth metals, ammonium, alkylammonium, alkanolammonium, glucoammonium corresponding to these acids: benzoic acid, sorbic acid, 4-methoxybenzoic acid, salicylic acid, undecylenic acid;
Organic alcohols and phenols: phenoxyethanol, benzyl alcohol, caprylyl glycol, ethylhexyl glycerolrine, phenethyl alcohol, 3-methyl-4-isopropyl phenol, 2,4-dichlorobenzyl alcohol;
Broad spectrum biocides: benzisothiazolinone, dodecyldipropylene triamine, methylisothiazolinone, mixture of these, sodium pyrithione, laurylamine dipropylenediamine and other commercially available components;
Fungicides: sodium pyrithione, climbazole and other commercially available components.

Enzymes: protease (ficin, bacillolysin, subtilisin), alpha-amylase, pectate lyase, mannanase, mannosidase, cellulase, amino oxidase, feruloyl esterase, beta-glucanase, tannase, alpha-glucosidase, beta-glucosidase, alpha-galactosidase, beta-galactosidase, manganese peroxidase, licheninase, xylanase and other commercially available enzymes used in laundry, dishwashing, floor, glass and all-purpose cleaners.

Bleaching agents based on oxygen compounds: hydrogen peroxide, calcium peroxide, carbamide peroxide, ε-phthalimidoperoxycaproic acid, sodium carbonate peroxide, TAED and other commercially available components.

Essential oil fragrances or essential oils in pure form or as blends in various proportions: orange, bergamot, lemon, lime, mandarin, grapefruit, neroli, rosewood, yuzu, lemongrass, lavender, sage, thyme, melissa, mint, tea tree, eucalyptus, cedar, sandalwood, black pepper, pink pepper, cinnamon, cardamom, coriander, jasmine, rose, peony, blue chamomile or other available essential oil.

In another aspect, the invention relates to the use of the laccase according to the invention for enzymatic cleavage of coloured melanoidins resulting from the Maillard reaction phenolic food compounds, hard-to-remove biocontaminants and biostains based on them on dense infections, safe of color and appearance. The use may be characterized in that said dense surface is selected from cotton, synthetic, blended, delicate (wool, silk, cashmere, merino, down, feather and their mixtures), membrane, teeth enamel based on hydroxyapatite.

### EXPERIMENTAL SECTION

The examples included in the present description are not intended to limit the claimed invention and are merely intended to illustrate and confirm the achievement of the expected technical results. These examples are among the many experimental data obtained by the authors of the invention which confirm the effectiveness of the means within the scope of the invention. With regard to the examples of the invention given below, it should be noted that a biolaccase having the CAS number CAS 80498-15-3 was used.

### Example 1

The laccase for inclusion in the composition according to the invention was investigated in various laundry detergents. A liquid detergent (formula No. 1), in particular a universal laundry detergent for white and coloured laundry, was prepared within the scope of the invention (Table 1).

**Table 1. Formulation of the liquid laundry detergent with the claimed laccase**

| | | |
|---|---|---|
| No. | Component | Content, wt.% |
| 1 | Purified water | up to 100.00 |
| 2 | Alkyl polyethylene glycol sulfate with the general formula R1-O(-CH2-CH2-O)n1(SO3)n2X1, wherein n1 takes a value from 0 to 10 and denotes the number of polyethylene groups, R1 represents an alkyl and/or alkenyl group with a hydrocarbon chain length of 5 to 22 carbon atoms, n2 takes a value from 0 to 1 and denotes number of sulfate groups, X1 represents alkali and/or alkaline earth metal cation, ammonium, alkylammonium, alkanolammonium, glucoammonium, basic amino acid | 1.5-10.00 |
| 3 | Salts of higher carboxylic acids with the general formula: R1-CO2X1, where R1 is an alkyl and / or alkenyl group with a long hydrocarbon chain from 5 to 21 carbon atoms, and X1 is an alkali and/or alkaline earth metal, ammonium, alkylammonium, alkanolammonium, glucoammonium cation, basic amino acid | 1.5-10.00 |
| 4 | Alkyl glucoside with the general formula: R28-O-[G]p3, wherein R28 is an alkyl and/or alkenyl group with a hydrocarbon chain length of 4 to 22 carbon atoms, G is a saccharide fragment containing 5 or 6 carbon atoms, p3 can take values from 1 to 4, in particular, C10-16 alkyl polyglucoside and C8-10 alkyl polyglucoside | 1.5-10.00 |
| 5 | Acylamidoalkylbetaine with the general formula: R18-C(O)-NH-R19-N(-CH3)2)-CH2-CO2, wherein R18 is an alkyl and/or alkenyl group with a hydrocarbon chain length of 5 to 21 carbon atoms, R19 is an alkyl group with a hydrocarbon chain length of 1 to 4 carbon atoms | 0.5-5.00 |
| 6 | Alkyl polyethylene glycol ether with the general formula: R29-O(-CH2-CH2-O-)n3H, where n3 can take values from 2 to 20, and denotes the number of polyethylene glycol groups, R29 is an alkyl and / or alkenyl group with a long hydrocarbon chain from 6 up to 22 carbons | 1.5-7.50 |
| 7 | Biolaccase | 0.03-1.00 |
| 8 | Bioenzymes such as protease, alpha-amylase, pectate lyase, lipase, mannanase or cellulase together or separately | 0.1-1.00 |
| 9 | Complexing agent | 0.1-1.00 |
| 10 | Complexing agent, corrosion inhibitor based on an inulin derivative | 0.1-1.00 |
| 11 | Glycerol of natural origin | 1.0-5.00 |
| 12 | Trinatrium citrate dihydrate | 0.5-3.00 |
| 13 | Sodium chloride | 1.0-5.00 |
| 14 | Cotton seed extract | 0.005-0.50 |
| 15 | Preservative | 0.005-0.75 |
| 16 | Sodium hydroxide or potassium hydroxide | 0.005-0.50 |
| 17 | Excipients if necessary | 2.0-25.00 |

Prepared liquid detergent for laundry washing and stain removal, once laccase has been added, is assumed to provide high efficiency of melanoidins and/or phenolic organic compounds breakdown in the biostains from various dense surfaces, in particular cotton, synthetic, membrane, linen fabrics, at any hardness of tap water of 0-15 °dH and at any washing temperature from +15 °C to +60 °C. It does not cause colour change and dye washout, retains the product's original appearance, leaves no streaks, is completely rinsed from the surface of fabric, suitable for washing children's laundry and people with sensitive hands, stable in storage for 24 months (observation time).

### Example 2

The laccase for inclusion in the composition according to the invention was investigated in various detergent formulations. A liquid laundry detergent (formula No. 2), in particular a liquid laundry detergent for coloured and white laundry and stain removal, was prepared within the scope of the invention (Table 2).

Formulation of the stain remover for washing coloured laundry with laccase

| No. | Component | Content, wt.% |
|---|---|---|
| 1 | Purified water | up to 100.00 |
| 2 | Alkyl polyethylene glycol sulfate with the general formula R1-O(-CH2-CH2-O)n1(SO3)n2X1, wherein n1 takes a value from 0 to 10 and denotes the number of polyethylene groups, R1 represents an alkyl and/or alkenyl group with a hydrocarbon chain length of 5 to 22 carbon atoms, n2 takes a value from 0 to 1 and denotes number of sulfate groups, X1 represents alkali and/or alkaline earth metal cation, ammonium, alkylammonium, alkanolammonium, glucoammonium, basic amino acid | 3.0-15.00 |
| 3 | Alkyl glucoside with the general formula: R28-O-[G]p3, wherein R28 is an alkyl and/or alkenyl group with a hydrocarbon chain length of 4 to 22 carbon atoms, G is a saccharide fragment containing 5 or 6 carbon atoms, p3 can take values from 1 to 4, in particular, C10-16 alkyl polyglucoside and C8-10 alkyl polyglucoside | 1.5-10.00 |
| 5 | Acylamidoalkylbetaine with the general formula: R18-C(O)-NH-R19-N(-CH3)2)-CH2-CO2, wherein R18 is an alkyl and/or alkenyl group with a hydrocarbon chain length of 5 to 21 carbon atoms, R19 is an alkyl group with a hydrocarbon chain length of 1 to 4 carbon atoms | 0.5-7.00 |
| 6 | Bioenzymes such as protease, alpha-amylase, pectate lyase, lipase, mannanase or cellulase together or separately | 0.1-1.00 |
| 7 | Biolaccase | 0.03-1.00 |
| 8 | Glycerol of natural origin | 0.0-5.00 |
| 9 | Sodium chloride | 1.0-5.00 |
| 10 | Cotton seed extract | 0.005-0.50 |
| 11 | Preservative | 0.005-0.75 |
| 12 | Sodium hydroxide or potassium hydroxide | 0.0-0.50 |
| 13 | Citric acid or another acid | 0.0-1.00 |
| 14 | Excipients if necessary | 0.5-10.00 |

Prepared liquid detergent for coloured and white laundry and stain removal, once laccase has been added, is assumed to provide high efficiency of melanoidins and/or phenolic organic compounds breakdown in the biostains from various dense surfaces, in particular cotton, synthetic, membrane, linen fabrics, at any hardness of tap water of 0-15 °dH and at any washing temperature from +15°C to +60°C. It does not cause colour change and dye washout, retains the product's original appearance, leaves no streaks, is completely rinsed from the surface of fabric, suitable for washing children's laundry and people with sensitive hands, stable in storage for 24 months (observation time).

### Example 3

The laccase for inclusion in the composition according to the invention was investigated in various detergent formulations. A dry detergent (formula No. 3), in particular a powdered detergent for washing coloured laundry and stain removal, was prepared within the scope of the invention (Table 3).

**Table 3. Formulation of the powdered detergent for washing coloured and white laundry with the laccase**

| No. | Component | Content, wt.% |
|---|---|---|
| 1 | Purified water | 2.0-7.50 |
| 2 | Alkylpolyethylene glycol ether with the general formula: R29-O(-CH2-CH2-O-)n3H, wherein n3 can take values from 2 to 20 and denotes the number of polyethylene glycol groups, R29 is an alkyl and/or alkenyl group with a hydrocarbon chain length of 6 to 22 carbon atoms | 1.0-10.00 |
| 3 | Alkyl carboxylate with the general formula R1-O(-CH2-CH2-O)n1 (SO3)n2 X1, wherein n1 takes a value from 0 to 10 and denotes the number of polyethylene groups, R1 represents an alkyl and/or alkenyl group with a hydrocarbon chain length from 5 to 22 carbon atoms, n2 is 0 to 1 and denotes the number of sulfate groups, X1 is an alkali and/or alkaline earth metal cation, ammonium, alkylammonium, alkanolammonium, glucoammonium, basic amino acid | 1.0-10.00 |
| 4 | Sodium carbonate | 10.0-40.00 |
| 5 | Sodium percarbonate | 5.0-20.00 |
| 6 | Sodium sulphate | 4.0-30.00 |
| 7 | Sodium silicate | 0.5-10.00 |
| 8 | Sodium aluminosilicate in the form of zeolites | 5.0-20.00 |
| 9 | Sodium aluminosilicate amorphous | 1.0-10.00 |
| 10 | Biolaccase | 0.03-1.00 |
| 11 | TAED | 0.5-5.00 |
| 12 | Lipase, mannanase, cellulase, amylase, protease | 0.1-2.00 |
| 13 | Carboxymethylcellulose sodium salt | 0.15-2.50 |
| 14 | Carboxymethylcellulose | 0.5-2.00 |
| 15 | Cotton seed extract | 0.005-0.50 |
| 16 | Excipients if necessary | 2.0-25.00 |

Prepared powdered detergent for washing coloured linen and stain removal, once laccase has been added, is assumed to provide high efficiency of breaking down melanoidins and/or phenolic organic compounds in the composition of biostains from various fabrics, in particular cotton, synthetic, membrane, linen, at any hardness of tap water of 0-15 °dH and at any washing temperature from +15°C to +60°C. It does not cause colour change and dye washout, retains the product's original appearance, leaves no streaks, is completely rinsed from the surface of fabric, suitable for washing children's laundry and people with sensitive hands, stable in storage for 36 months (observation time).

### Example 4 (not part of the present invention)

A toothpaste (formula No. 4), in particular a whitening toothpaste, was prepared (Table 4).

**Table 4. Formulation of the toothpaste for cleaning and whitening with the laccase**

| No. | Component | Content, wt.% |
|---|---|---|
| 1 | Purified water | up to 100,00 |
| 2 | Thickener natural and/or synthetic | 0.1-10.00 |
| 3 | Silicon dioxide | 1.0-30.00 |
| 4 | Solvent | 1.0-70.00 |
| 5 | Surfactant | 1.0-15.00 |
| 6 | Preservative | 0.005-3.00 |
| 7 | Flavoring agent | 0.005-3.00 |
| 8 | Sweetener | 0.005-1.00 |
| 9 | Excipients if necessary | 2.0-25.0 |

### Example 5

A laboratory study of laccase was carried out to quantify the degradation of melanoidins and phenolic organic compounds. The study was carried out in Erlenmeyer flasks (75 ml) containing 10 ml of pre-freshly prepared solution of coffee beans. Freshly prepared espresso was prepared in a coffee machine, a standard portion of 80 ml, then diluted with distilled water 200 times to the initial coffee pH in the range of 5.64-5.80. The coffee solution was then adjusted to the desired pH of 3.0 with 1M hydrochloric acid. NaOH/HCl to assess laccase activity in the desired pH range. A 0.01% hydrogen peroxide solution was used as an active control (this % input did not interfere with UV spectrophotometry). pH 3.0 was chosen for a fair comparison with hydrogen peroxide and is consistent with oxygen stain removers for bleaching stains. Then 0.25 wt.% laccase raw material (in terms of the active ingredient 0.0875 wt.% laccase) was added from various fungi (Table 5) to select the optimal raw material. Flasks with coffee solutions after addition of enzymes were incubated at 40 ± 1°C under conditions of shaking the flask (180 rpm) for 60 min. The degradation of melanoidins and phenolic organic compounds was assessed by measuring the absorption maxima of melanoidins and phenolic organic compounds (POC) at 325 nm (due to the presence of caffeine in the composition of coffee beans) using a UV-Vis spectrophotometer, measuring range 200-700 nm. At the same time, a flask containing only coffee solution adjusted to the required pH was used as a control. The percentage degradation of selected compounds by UV spectrometry was calculated by the formula: 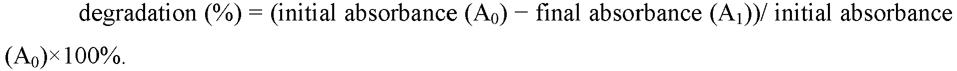 degradation (%) = (initial absorbance (A0) - final absorbance (A1))/ initial absorbance (A0)x 100%.

**Table 5. Laccases from various sources**

| 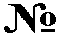 | **Laccase** | | **Source** | **Activity** | **Optimal pH** | **Optimal temperature** |
|---|---|---|---|---|---|---|
| 1 | Laccase 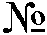 1 | | *White rot fungi* | 10 U/g | 3.0-5.5 | 20-60°C |
| 2 | Laccase 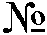 2 | | *Trametes versicolor* | ≥ 0.5 U/mg | 4.0-6.5 | 20-60°C |
| 3 | Laccase 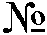 3 | | *Pleurotus ostreatus* | >380 U/g | 4.5-6.5 | 30-65°C |
| 4 | Laccase 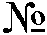 4 | | *Aspergillus oryzae* | >10000 U/g | 3.5-6.0 | 20-60°C |

### Results.

Based on the results of assessing the degradation of melanoidins and phenolic organic compounds, it was found that laccase 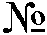 4 from Aspergillus oryzae showed optimal activity (Table 6). The active control, hydrogen peroxide solution, showed low degradation of melanoidins and phenolic organic compounds. Laccase 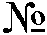 3 showed an increase in the optical density of solutions, which indicates their darkening and the formation of colored products that can potentially be fixed on dense surfaces and are more difficult to remove. Laccase 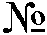 1 had a high activity against phenolic organic compounds (OPC) and coffee melanoidins, but had a low water solubility, which is not applicable for final household detergents and oral care products. Laccase 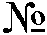 2 had low activity against melanoidins and FOS, which are common in the composition of complex melanoidins, for example, chlorogenic acid and its isomers. It should be noted that laccase 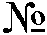 2 must be stored at a temperature of -18°C, which is difficult to implement under industrial conditions and causes low enzyme stability in the finished household detergent under room storage conditions. The source of laccase has been shown to be important, as the chosen strain of fungus influences the final potency of laccase and its properties, such as pH sensitivity and pH range, stability over a wide temperature range and in the presence of various basic excipients or additives.

**Table 6. The results of the study of laccase and hydrogen peroxide against coffee organic compounds by UV spectrophotometry**

| 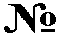 | **Sample** | **Degradation of melanoidins and phenolic organic compounds, %** |
|---|---|---|
| 1 | Laccase from *White rot fungi -* 0.25 wt.% laccase raw material (in terms of the active ingredient 0.0875 wt.% laccase) | -28.5% |
| 2 | Laccase from *Trametes versicolor -* 0.25 wt.% laccase raw material (in terms of the active ingredient 0.0875 wt.% laccase) | -9.1% |
| 3 | Laccase from *Pleurotus ostreatus -* 0.25 wt.% laccase raw material (in terms of the active ingredient 0.0875 wt.% laccase) | +45.4% |
| 4 | Laccase from *Aspergillus oryzae -* 0.25 wt.% laccase raw material (in terms of the active ingredient 0.0875 wt.% laccase) | -30.4% |
| 5 | 0.01% hydrogen peroxide solution | -6.0% |

Since sample of laccase 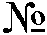 4 had optimal activity, the degradation of melanoidins and phenolic organic compounds was quantified at pH of 7.0, which is outside the optimum activity of the enzyme according to technical data. The study was carried out by the method described above. The results of the study are shown in Table 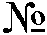 7. It has been shown that laccase from the fungi Aspergillus spp., in particular Aspergillus oryzae, retains its activity both at acidic pH and at neutral pH, which is important for use in household detergents against stubborn stains. The pH shift to the neutral region increases the degradation of coffee melanoidins and phenolic organic compounds by laccase by +5.6% (difference in degradation), reaching a value of 36.0% degradation at neutral pH.

**Table 7. Results of the study of laccase from Aspergillus oryzae at pH of 3.0 and 7.0**

| **pH** | **Sample** | **Degradation of melanoidins and phenolic organic compounds, %** |
|---|---|---|
| 3,0 | Laccase 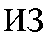 Aspergillus oryzae - 0.25 wt.% laccase raw material (in terms of the active ingredient 0.0875 wt.% laccase) | -30.4% |
| 7,0 | Laccase 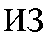 Aspergillus oryzae - 0.25 wt.% laccase raw material (in terms of the active ingredient 0.0875 wt.% laccase) | -36.0% |

The ability of laccase to degrade phenolic and non-phenolic compounds, such as melanoidins, significantly expands the area of potential application of the enzyme in household detergents and oral care products. Simple and complex melanoidins are a group of heterogeneous substances that are widely distributed among food products: in coffee, cocoa beans, chocolate, bakery products, dark beer, wine, nuts and seeds, soy sauce, balsamic vinegar and other products that leave stains that are difficult to remove dense surfaces. The use of laccase as a bioenzymatic bleach makes it possible to remove complex stains at home, while maintaining the color of dense surfaces, their structure and their appearance. Laccase 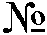 4 from Aspergillus oryzae turned out to be optimal in terms of effect with respect to both groups of organic compounds - melanoidins and FOS, so further studies were carried out with it.

### Example 6

A laboratory study of laccase was carried out to qualitatively assess the degradation of melanoidins and phenolic organic compounds and to prove a targeted effect on both groups of organic compounds. Erlenmeyer flasks (75 ml) were taken, 15 ml of pre-freshly prepared diluted coffee bean solution (espresso) was added. Freshly prepared espresso was prepared in a coffee machine, a standard portion of 80 ml, then diluted with distilled water 200 times to the initial coffee pH in the range of 5.64-5.80. The coffee solution was then adjusted to the desired pH of 3.0 with 1M hydrochloric acid HCl to assess laccase activity. Then 0.25 wt.% laccase raw material (in terms of the active ingredient 0.0875 wt.% laccase) from Aspergillus spp., in particular Aspergillus oryzae, was added. Flasks with coffee solutions after addition of enzymes were incubated at 40 ± 1°C under conditions of shaking the flask (180 rpm) for 60 min. The degradation of melanoidins and phenolic organic compounds was assessed using a chromato-mass-spectrometric study. The method of chromato-mass spectrometry makes it possible to identify the main compounds of destruction of melanoidins and coffee. Samples were analyzed using a Nexera HPLC system (Schimadzu) consisting of a pump (LC-30AD), an autosampler (SIL-30AC), a column thermostat (CTO-20A), and a diode array detector (SPO-M20A). For chromatographic separation, an Explice Plus C18 analytical column with a size of 3x10 mm and a particle diameter of 3.5 µm was used. Chromatographic separation was carried out in a gradient mode using a mixture of acetonitrile (A) and water (B) with the addition of 0.1% formic acid as a mobile phase. Gradient program: linear increase in acetonitrile concentration from 10 to 100% from 0 to 15 minutes, hold at 100% A from 15 to 16 minutes, return to baseline from 16 to 17 minutes, hold at 10% A from 17 to 18 minutes. Flow rate 0.4 ml/min, column temperature control at 40°C. Detection was carried out on a diode array detector (DAD) in the range from 190 to 800 nm.

The compounds were identified using a QExactive Plus mass spectrometric system. The analytes were ionized by electrospray (ESI). Detection was carried out in the mode of registration of negatively charged ions in the m/z range of 100-1500 Da.

### Results.

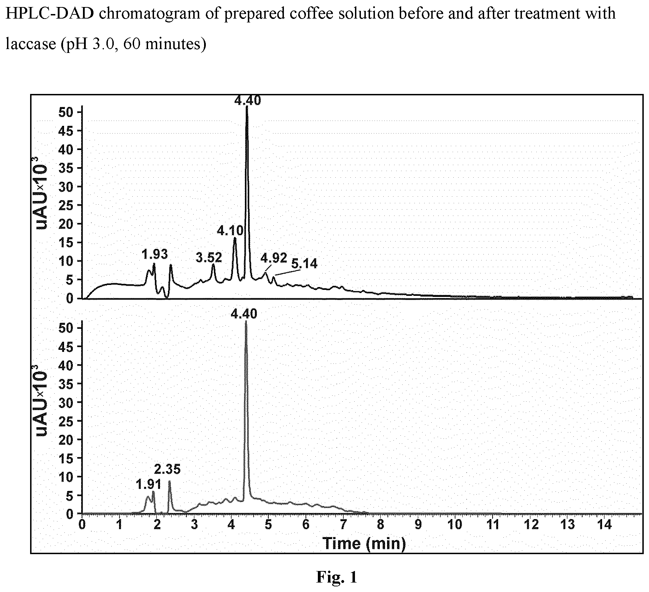

Based on the HPLC-DAD results, the following chromatograms were obtained, shown in Fig.1. The main difference in the chromatograms is a significant decrease in the intensity of analytical signals for peaks with a retention time of 3.52, 4.10, 4.92, 5.14 min, which indicates the degradation of simple and complex melanoidins. A characteristic peak with a retention time of 4.40-4.42 corresponds to the main substance of coffee - caffeine, which is colorless and does not stain dense surfaces, but is present in large quantities in coffee beans.

Compounds were identified by high resolution mass spectrometry. The list of identified compounds is presented in Table 8. According to the results of the study, melanoidin degradation products of various structures were identified after treatment with laccase, which indicates the targeted effect of laccase on melanoidins and phenolic organic compounds, in particular, isomers of chlorogenic acid, which are part of melanoidins. Also, as a result of the study, the role of saccharides in the formation of melanoidins as glycosylated products was confirmed. Additionally, the change in content after treatment of melanoidins and phenolic organic compounds with laccase was evaluated (Table 9). It was found that the concentration of simple and complex melanoidins with phenolic organic compounds decreases from 42.9% to 73.5% after treatment with laccase, which confirms the high efficiency of this enzyme and the targeted action.

**Table 8. Identification of the main compounds in coffee after treatment with laccase**

| tr, min | m/z, Da | [M+H]+ | [M-H]- | Compounds |
|---|---|---|---|---|
| 3.52 | 355.1026 | C₁₆H₁₉O₉ | - | isomer of chlorogenic acid, simple melanoidin |
| 4.10 | 355.1026 | C₁₆H₁₉O₉ | - | isomer of chlorogenic acid, simple melanoidin |
| 4.42 | 195.0876 | C₈H₁₁O₂N₄ | - | caffeine |
| 4.92 | 355.0775 | - | C₁₆H₁₅O₈ | 3-Caffeoyl-1,5-quinolactone isomer, complex melanoidin |
| 5.14 | 355.0775 | - | C₁₆H₁₅O₈ | 3-Caffeoyl-1,5-quinolactone isomer, complex melanoidin |
| 6.76 | 268.1181 | C₁₁H₁₆O₄N₄ | - | non-glycosylated degradation products of melanoidins |
| 7.17 | 222.1135 | - | C₁₂H₁₆O₃N | |
| | 266.1033 | | C₁₃H₁₆O₅N | |
| 8.10 | 270.1134 | - | C₁₆H₁₆O₃N | |
| | 314.1034 | | C₁₇H₁₆O₅N | |

**Table 9. Change in the content of melanoidins in coffee after treatment with laccase**

| [M+H]+ | [M-H]- | Compounds | Intensity | | % of degradation |
|---|---|---|---|---|---|
| | | | Before | After | |
| W₁₆H₁₉O₉ | - | isomer of chlorogenic acid, simple melanoidin | 10000 | 3000 | -70.0% |
| W₆H₁₉O₉ | - | isomer of chlorogenic acid, simple melanoidin | 17000 | 4500 | -73.5% |
| - | C₁₆H₁₅O₈ | 3-Caffeoyl-1,5-quinolactone isomer, complex melanoidin | 7000 | 3000 | -57.1% |
| - | C₁₆H₁₅O₈ | 3-Caffeoyl-1,5-quinolactone isomer, complex melanoidin | 6000 | 3000 | -50.0% |
| C₁₁H₁₆O₄N₄ | - | non-glycosylated degradation products of melanoidins | 7000 | 4000 | -42.9% |
| - | C₁₂H₁₆O₃N | | | | |
| | C₁₃H₁₆O₅N | | | | |
| - | C₁₆H₁₆O₃N | | | | |
| | C₁₇H₁₆O₃N | | | | |

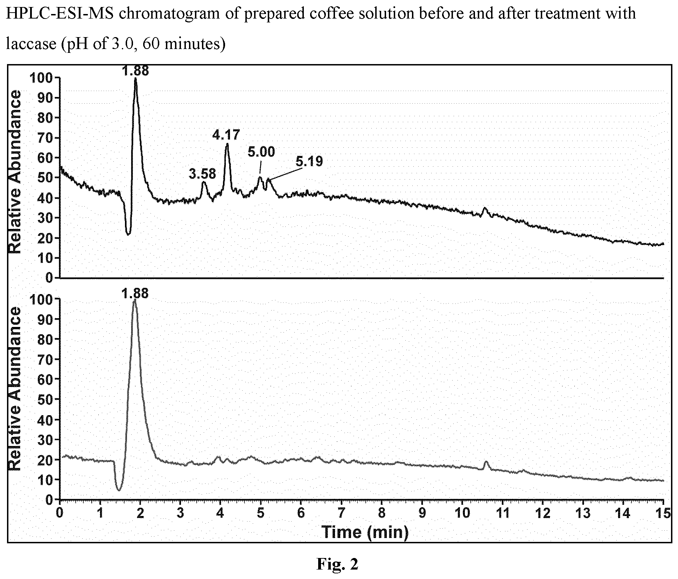

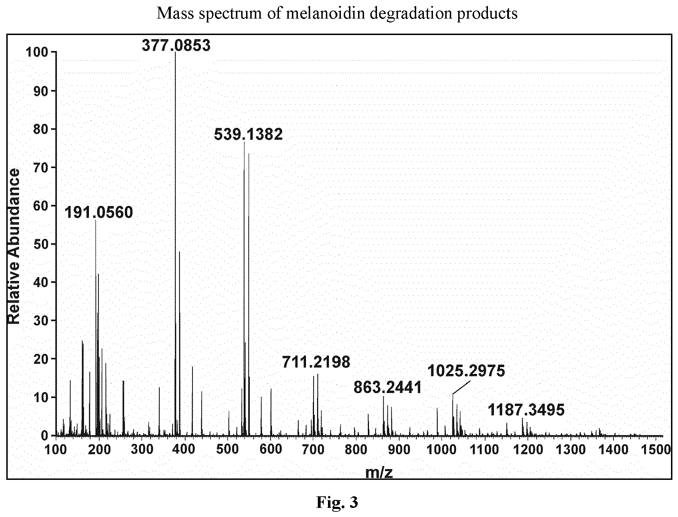

According to the results of HPLC-ERI-MS, the following chromatograms were obtained, presented in Fig.2. The key feature of these chromatograms is the appearance of an intense peak at 1.88 minutes in the chromatogram of the laccase-treated coffee sample. The mass spectrum for this peak (Fig. 3.) showed the presence of successive ions with m/z 215, 377, 539, 701, 863, 1025, 1187 differing by the same link 162, which apparently corresponds to the structure of hexoses (C₆H₁₂O₆, M=180 Da), which split off 1 molecule of water. A similar picture is observed for coffee treated with laccase at pH of 7.0 and 60 minutes of treatment.

Thus, chromato-mass-spectrometric analysis of the presented coffee samples before and after enzymatic treatment with laccase showed targeted degradation of melanoidins of various structures and phenolic organic compounds. A decrease in the secondary components (chlorogenic acid and its compounds) has been shown that make up coffee after enzymatic treatment with laccase. During the study, melanoidin degradation products were found in coffee samples after enzymatic treatment with laccase, which indicates the destruction of these compounds, and which was quantitatively confirmed in example 4 by UV spectrophotometry. It has been established monosaccharides of the hexose type play a key role that in the formation of high-molecular-weight melanoidin in this sample of coffee, which are cleaved off during the enzymatic action of laccase on coffee.

### Example 7

A laboratory study of laccase (Aspergillus oryzae) at various concentrations was carried out to assess the degradation of melanoidins and phenolic organic compounds. The aim of the study was to determine the range of effective concentrations of the selected laccase for a high target action against melanoidins and phenolic organic compounds. The study was carried out according to the procedure described in Example 5 at pH of 3.0 and 7.0 to confirm the pH stability of laccase and its effect. Laccase was added at concentrations of 0.10, 0.25, 0.50, 0.75 and 1.00 wt.% laccase raw material (in terms of the active ingredient 0.03, 0.0875, 0.175, 0.263 and 0.350 wt.% laccase). The exposure time was 60 minutes, which corresponds to the average wash cycle time of various dense surfaces. The degradation of melanoidins and phenolic organic compounds was assessed by measuring the absorption maxima of melanoidins and phenolic organic compounds (POC) at 325 nm (due to the presence of caffeine in the composition of coffee beans) using a UV-Vis spectrophotometer, measuring range 200-700 nm. At the same time, a flask containing only coffee solution adjusted to the required pH was used as a control. The percentage degradation of selected compounds by UV spectrometry was calculated using the formula given in Example 5.

### Results.

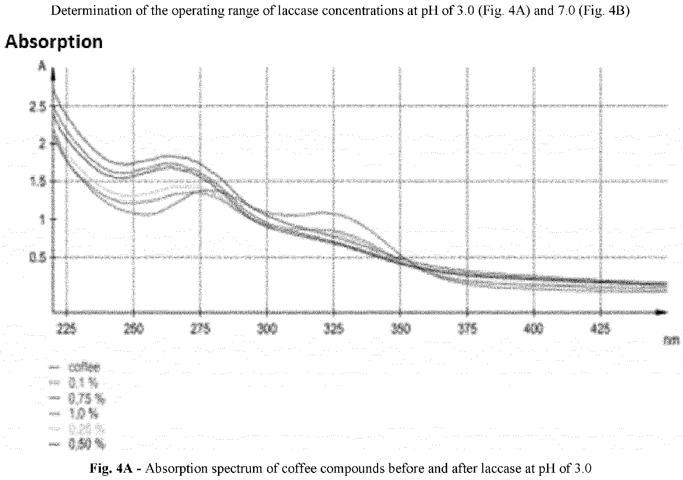

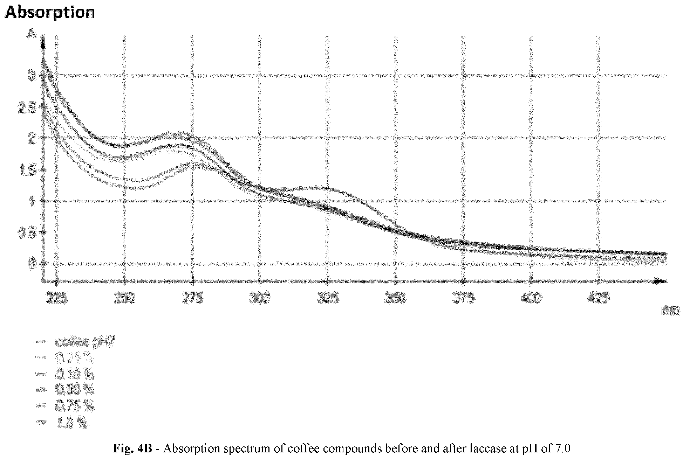

Based on the results of assessing the degradation of melanoidins and phenolic organic compounds, it was found that the optimal concentration of laccase is in the range from 0.10% to 0.75 wt.%. Absorption spectra (Fig. 4) show that laccase degrades melanoidins and phenolic organic compounds (absorption maximum 330 nm), both at pH of 3.0 and at pH of 7.0 relative to the original coffee sample (red spectrum). At pH of 3.0, the optical density of melanoidins decreases in proportion to the enzyme concentration, with the optimal laccase concentration from 0.030% to 0.263%, and a further increase in the enzyme content does not lead to a decrease in the optical density in the region of 300-350 nm. The greatest effect is achieved in the range of 0.25 to 0.50 wt.% laccase, more precisely at 0.50 wt.% (in terms of the active ingredient 0.175 wt.% laccase). At a concentration of 1.00 wt.%, there is a reverse increase in the absorption spectrum at 325 nm, which can be negative in the case of removing melanoidin stains from various sources on dense surfaces. At pH 7, the optical density reaches its minimum value already with the addition of laccase at a concentration of 0.10 to 0.25 wt.%, or rather, at 0.25 wt.% (in terms of the active ingredient 0.0875 wt.% laccase).

The ability of laccase to decompose phenolic and non-phenolic compounds, such as melanoidins, significantly expands the area of potential application of the enzyme in household detergents and oral care products. Simple and complex melanoidins are a group of heterogeneous substances that are widely distributed among food products: in coffee, cocoa beans, chocolate, bakery products, dark beer, wine, nuts and seeds, soy sauce, balsamic vinegar and other products that leave stains that are difficult to remove. dense surfaces. The use of laccase as a bioenzymatic bleach makes it possible to remove complex stains at home, while maintaining the color of dense surfaces, their structure and their appearance.

### Example 8

A laboratory study of laccase (Aspergillus oryzae) and protease (subtilisin from Bacillus subtilis) was carried out to assess the interaction of enzymes with the joint presence and activity of both enzymes. Laccase raw material 0.25 wt.% (in terms of the active ingredient 0.0875 wt.% laccase) was used for research. The test was conducted in the laboratories of the Sunson Enzymes Group using their own methods to determine the activity of enzymes individually and in the presence of a joint. This test evaluates the retention of enzyme activity at various pH levels in order to determine the residual activity over the life and shelf life and therefore the effectiveness of the product with both enzymes.

Guaiacol was used as a substrate for laccase. 1 unit of laccase activity corresponded to the amount of enzyme required to oxidize 1 mole of guaiacol in 1 minute at 30°C and at the chosen pH. The guaiacol oxidation product was evaluated by UV spectrophotometry at 465 nm, then the concentration was calculated using a special mathematical formula: $X = ΔA \times n \times \left(3\div \left(0,6\times 12000\times 30\right)\right) \times 1000000$

In the formula:
X-Enzyme activity, unit is µmol/ml(g).min
△A-Absorbance, measuring at 465nm
n-Dilution ratio
3-Total reaction volume (mL) :
0.6-Volume of sample in the reaction (mL) :
12000-Molar extinction coefficient;
30-Reaction time (min) :
1000000-Conversion ratio of µmol and mol

The results of the study of enzyme activity in parallel determinations should be reproducible and not differ by more than +10.0%. The tests were carried out three times (n=3).

### Results.

Based on the results of the assessment of the residual activity of enzymes, it was found that the studied biolaccase and bioprotease retain their activity separately and in the joint presence in a wide pH range (Table 10). This fact indicates the possibility of the joint presence of two enzymes in the recipes of household detergents and the preservation of high efficiency values, demonstrated further in Examples 9-10.

**Table 10. Residual activity of the enzymes of the composition at different pH**

| **Tested sample** | **Components** | **Initial laccase activity** | **Residual enzyme activity, %** | |
|---|---|---|---|---|
| | | | **pH 3.5** | **pH 7.0** |
| Sample 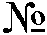 1 | Laccase 0.25 wt.% | 10000 U/g | 10000 U/g | 10000 U/g |
| Sample 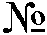 2 | Laccase 0.25 wt.% in presence of protease (subtilisin) 0,0125% a.e.p. | | 10735 U/g | 7150 U/g |

Laccase in the presence of a protease (subtilisin) retains its activity over a wide pH range to achieve high removal rates for a wide range of biostains and biocontaminants based on melanoidins and phenolic organic compounds on dense surfaces. Since laccase is not stable in the presence of protease according to the literature, the above results indicate a new technical result achieved by the characteristics of the raw material, and the possibility of joint use to achieve the claimed effects of the composition according to the invention.

### Example 9 (not part of the present invention)

A laboratory study of the detergent effectiveness of the laccase as part of a universal stain remover for color and white fabrics was carried out. As a base for introducing the laccase, a liquid universal stain remover for white and coloured laundry was used, as defined in Table 2.

The test methodology is based on the generally recognised recommendations of the European Association AI.S.E. [A.I.S.E. Laundry Detergent Testing Guidelines Minimum requirements for comparative detergents testing, see https://www.aise.eu/documents/document/20180625164030-laundry_detergent testing_guidelines_v_5_2_june_2018_pdf, to determine the detergent efficacy with regard to stain removal index (SRI) and the values L,a,b. The SRI is calculated using the formula from the ASTM D 4265 Standard guide for evaluating stain removal performance in home laundering [DOI: 10.1520/D4265-14]. This guide was prepared by the American Society for Testing and Materials (ASTM) Committee D-12 on soaps and other detergents and was published in 2014. This test evaluates the effectiveness of the composition in removing fresh and stale stains of various nature from cotton and synthetic materials simulating actual washing conditions. Washing was carried out in a Bosch WAB 24272 CE washing machine. Common recommendations in the European Union and Russian Federation were chosen as test conditions, in particular a temperature of 40 °C, a water hardness of 10.9 °dH and a standard wash mode (mixed fabrics). Three repetitions (n=3) were carried out, and the deviation was no more than 0.18% for each sample.

Hard-to-remove biostains based on melanoidins and phenolic organic compounds were used for the test (Table 11). The selected stubborn biostains allowed the overall detergent efficacy of the formulation to be assessed, as well as the efficacy against individual contaminants due to the presence of the composition of the invention.

**Table 11. Biostains and biocontaminants tested from A.I.S.E.**

| **Designation** | **Composition** | **Type** |
|---|---|---|
| EMPA 106 | mineral oil/soot | pigment-oil-based, dispersible |
| EMPA 118 | skin grease/pigment | pigment-oil-based, dispersible |
| EMPA 116 | blood/milk/ink | mixed: protein + pigment |
| CFT CS-44 | chocolate drink | mixed: protein + bleach* |
| CFT CS-08 | grass | mixed: protein + bleach* |
| CFT CBC-01 | tea | bleachable* |
| EMPA 114 | red wine | bleachable* |
| CFT CS-12 | blackcurrant juice | bleachable* |
| CFT CBC-02 | coffee | bleachable* |
| *this stain contains simple or complex melanoidins and/or phenolic organic compounds | | |

2-3 ml of distilled water, and then 2 ml of stain remover were applied to samples of standard soils (55x45 mm in size), fixed on a dense white cotton fabric, placed on a flat surface of the table, and evenly distributed over the surface of the pollution (5 circular motions). clockwise + 5 circular movements counterclockwise, simulating the washing process in everyday life) with a hard plastic brush. After 10 min, the washing operation was repeated on each contamination. Total prewash time 15 min. Then the treated soil samples were transferred to the washing machine and washed using 50 g of the universal liquid detergent indicated in Example 1. For a comparative assessment of the washing results, a similar washing was carried out in parallel with the same soiled samples without pretreatment, and household detergents were also taken from the market.

SRI is a quantitative measure of the removal of dirt and stains in units. The values are based on whiteness L and chromaticity a,b, calculated mathematically using a special formula from ASTM D 4265-14. The method has numerous advantages, in particular, high accuracy, reproducibility, low error rate (less than 5%), takes into account colours of laundry and their change in the process of washing, different types of fabric and is the closest to the real visual perception and balances out the effect of optical dyes, common in detergents for laundry washing. A difference of 2 or more SRI units is effective and statistically significant. In practical experience, increased 1 unit of SRI corresponds to 5% increased detergent efficacy and gives a significant contribution to the formulation.

### Results.

According to the results of evaluation of effectiveness and removal rate of stubborn biostains based on melanoidins and phenolic organic compounds, it was established that the studied laccase in the liquid universal stain remover for white and colored laundry has a strong stain-removing effect in respect of complex soiling of coffee, red wine, chocolate and other stains based on coloured melanoidins and/or phenolic organic compounds, compared to the comparison means, not containing the laccase according to the invention.

At the end of the study, marked changes were observed in the assessed index of removal efficiency of stubborn biostains based on melanoidins and phenolic organic compounds on dense surfaces. According to the dynamics of the index, the addition of high performance biolaccase increased the degree of removal of melanoidins stains. The addition of 0.25 wt.% laccase raw material (in terms of the active ingredient 0.0875 wt.% laccase) increased the SRI index by 48.8 units compared to no pretreatment and washing with laundry gel (statistically significant, p<0.05). The universal stain remover with laccase was effective against coloured stains with melanoidins and phenolic organic compounds, blood, grass and chocolate. The laccase allowed to significantly increase the stain-removing efficiency as part of a universal concentrated stain remover for washing white and colored laundry. An active oxygen (hydrogen peroxide) based stain remover sample is a common stain remover and bleach, but has not been shown to be very effective, especially on melanoidin and/or phenolic acid-based stains such as chocolate drink, grass, coffee, and blood stains.

The base sample without the laccase of the invention did not show a very high removal of the selected biostains, indicating a lack of efficacy of the surfactants in removing the soiling during laundry and the need for pretreatment (Table 11).

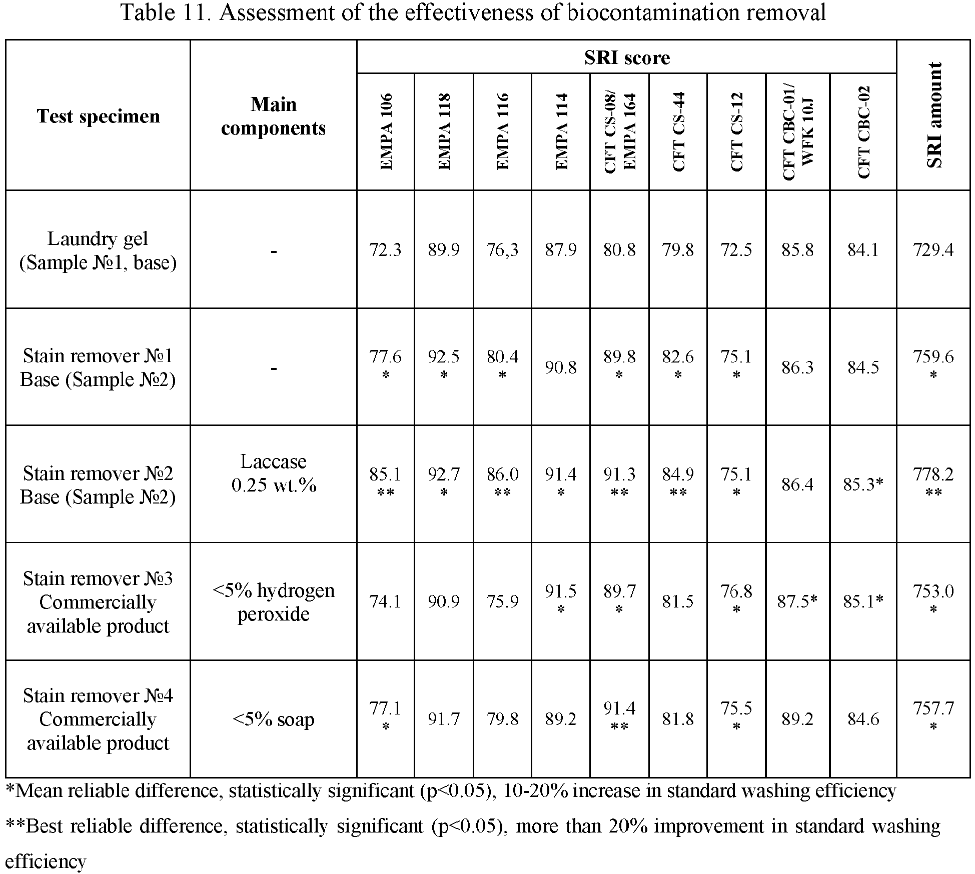

The authors have unexpectedly found that laccase is incompatible with complexing agents, for example, tetrasodium salt of glutamic acid, and it is necessary to maintain a certain pH range (Table 12). The presence of a complexing agent has been shown to reduce the effectiveness of laccase against melanoidin and/or phenolic organic acid stains, in particular grass, wine, coffee, and blood and motor oil. Often the effectiveness of stain removers is provided by alkaline pH, however, this is undesirable for dense surfaces due to their damage.

Lowering the pH to the neutral zone in the presence of a complexing agent further reduces the effectiveness of removing stains based on melanoidins and/or phenolic organic acids. Stabilization at pH of 7.0-8.0 and the absence of a complexing agent can significantly increase the effectiveness of the stain remover with laccase in relation to all types of stains by 48.8 SRI units, which indicates the pH sensitivity of biotechnology based on laccase for the degradation of melanoidins and/or phenolic organic compounds on dense fabrics.

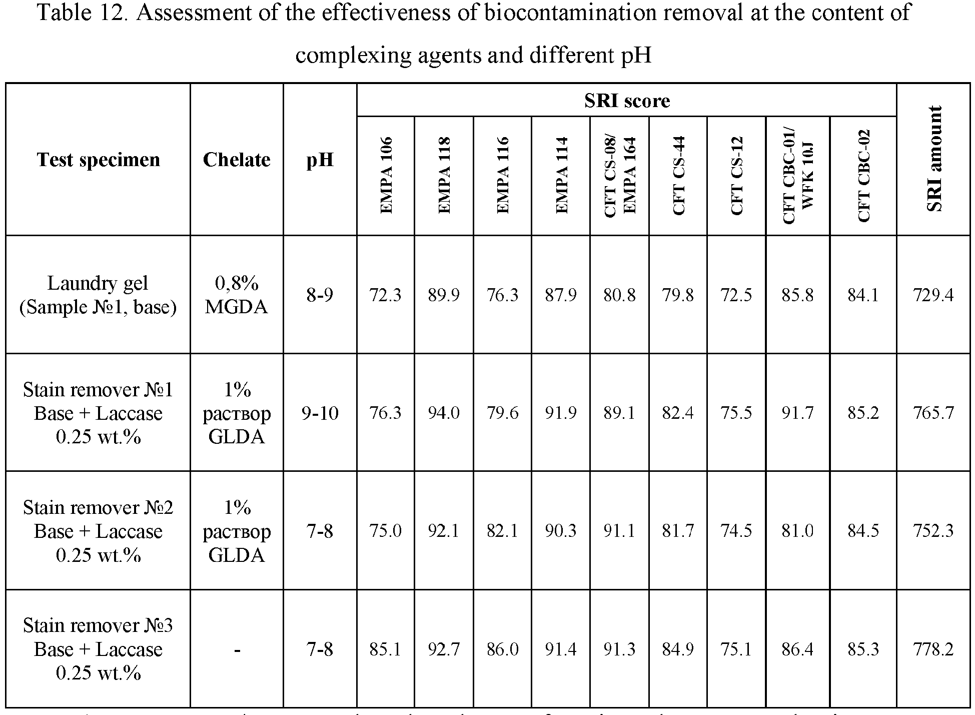

A consumer study was conducted on the use of a universal concentrated stain remover for washing white and colored laundry with laccase by application to evaluate overall impressions, cleaning products and difficult stains, color safe after product use. There are 12 people involved in the consumer experience. The terms of use were the same as described above. The 42% of consumers noted that there are stains of fruits, berries, wines, tea, coffee, cocoa, chocolate on their fabrics. 84.6% of the stains were the most recent, and 15.4% of the stains were old and stubborn.

According to a consumer study, 66% of consumers gave the highest score for the effectiveness of removing stains of various nature. Consumers noted that the stain remover easily coped with coffee, chocolate, fruit and other stains, quickly forming foam on the fabric and starting to act from the first minute. 100% of consumers noted the preservation of the brightness of the color and structure of fabrics, which indicates a soft effect on the fabric and the preservation of its appearance (Table 13).

**Table 13. Results of consumer test of stain remover with laccase**

| **Index** | **Index after use, max 3 points** | **% of consumers who gave the highest score** | **Comments** |
|---|---|---|---|
| Stain removal and performance test | 2.67±0.49 | 66.7% | Effective removal of fresh and old stains, stain remover quickly forms foam. |
| Color brightness | 3.00±0.00 | 100.0% | No damage to fabric color, no color washout or transfer after use. |
| Preservation of the shape and structure of fabrics | 3.00±0.00 | 100.0% | No damage to the fabric structure, no pilling. |

Complex biological contaminants based on melanoidins and/or phenolic organic acids are difficult to remove during the washing process due to fixation in hard-to-reach places and fibers of various dense surfaces and insufficient action of surfactants. A high content of surfactants increases the residual amount of substances on the fibers of the fabric, generates a large amount of foam and drastically reduces the wear resistance of the fabric, which can negatively affect the environment, increase water consumption, reduce washing performance, and significantly increase fabric wear and reduce color brightness.

The composition of a universal concentrated stain remover for washing white and colored laundry with laccase allows you to effectively remove dirt and stains from dense surfaces and hard-to-reach areas of various, thereby reducing the total amount of surfactants in household detergents, maintaining dermatological safety for humans and achieving almost complete removal of biological stains on based on simple and complex melanoidins. The removal of melanoidins and/or phenolic organic compounds provides a hygienic function, prevents excessive sorption of the contaminant on the surface of the fabric and reduces the number of washes in the long term. An increase in SRI after stain pre-treatment demonstrates improved stain removal efficiency, high light refractive power of the fabric, as well as the brightness of fabrics. The addition of laccase can improve the efficiency of removing food stains (chocolate drink, cocoa, coffee, grass, fruit stains) and blood stains without compromising the appearance of dense surfaces.

### Example 10 (not part of the present invention)

A laboratory study of color retention using the CRI color rendering index was carried out. A liquid concentrated stain remover for coloured and white fabrics was used as a base for introducing laccase, as shown in Table 2.

The test methodology is based on the generally recognised recommendations of the European Association A.I.S.E. [A.I.S.E. Laundry Detergent Testing Guidelines Minimum requirements for comparative detergents testing, see https://www.aise.eu/documents/document/20180625164030-laundry detergent testing guidelines v 5 2 june 2018 .pdf], to determine the brightness of a color, taking into account the color rendering index (CRI) and L,a,b values for the full spectrum of RGBW (red, green, blue, white) light. The CRI is calculated as SRI using the formula from ASTM D 4265 Standard guide for evaluating stain removal performance in home laundering [DOI: 10.1520/D4265-14] with some modifications: $CRI = 100 - {\left[{\left({L}_{c}-{L}_{w}\right)}^{2}+{\left({a}_{c}-{a}_{w}\right)}^{2}+{\left({b}_{c}-{b}_{w}\right)}^{2}\right]}^{1 / 2} ,$ where
L - fabric reflectivity or lightness, a - ratio red/green, b - ratio yellow/blue, c - washable fabric, w - unwashed fabric.

This test evaluates the effectiveness of the laccase in terms of the degree of retention of color brightness and the correct perception of color shades in the daytime and evening from dense surfaces. Washing was carried out in a Bosch WAB 24272 CE washing machine. Common recommendations within the European Union and Russian Federation were used as test conditions, in particular a temperature of 40°C, a water hardness of 10.9 °dH and a standard wash cycle (mixed fabrics). Three repetitions (n=3) were carried out, and the deviation was no more than 0.18% for each sample. For testing, various dense surfaces were used, in particular dense cotton fabrics in pink and blue (Table 14).

**Table 14. Tested coloured fabrics**

| **Name** | **Composition of fabrics** | **Initial CRI score** |
|---|---|---|
| Blue cotton | 100% cotton | 100.0 |
| Pink cotton | 100% cotton | 100.0 |

2-3 ml of distilled water, and then 2 ml of stain remover were applied to samples of standard soils (55x45 mm in size), fixed on a dense white cotton fabric, placed on a flat surface of the table, and evenly distributed over the surface of the pollution (5 circular motions). clockwise + 5 circular movements counterclockwise, simulating the washing process in everyday life) with a hard plastic brush. After 10 min, the washing operation was repeated on each contamination. Total prewash time 15 min. Then the treated soil samples were transferred to the washing machine and washed using 50 g of the universal liquid detergent indicated in Example 1. For a comparative assessment of the washing results, a similar washing was carried out in parallel with the same soiled samples without pretreatment, and household detergents were also taken from the market.

CRI is a quantitative measure of the color brightness in units. The values are based on whiteness L and chromaticity a,b, calculated mathematically using a special formula from ASTM D 4265-14 as SRI. The method has numerous advantages, in particular, high accuracy, reproducibility, low error rate (less than 5%), takes into account colours of laundry and their change in the process of washing, different types of fabric and is the closest to the real visual perception and balances out the effect of optical dyes, common in detergents for laundry washing. A difference of 2 or less CRI units is effective and statistically significant in color retention.

### Results.

Based on the results of assessing the brightness of the color of dense cotton fabrics, it was found that the universal concentrated stain remover for washing white and colored laundry with laccase in the composition retains the brightness of the original color and does not wash it out after preliminary mechanical treatment. At the end of the study, there was observed the preservation of color brightness and a slight change in the color rendering index CRI, due to the mechanical action of the stain remover on standard cotton fabrics. The addition of 0.25 wt.% laccase raw material (in terms of the active ingredient 0.0875 wt.% laccase) kept the CRI within the margin of error, which would be indistinguishable to the eye and the user would not notice a difference in color change (Table 15).

**Table 15. Evaluation of the retention of color brightness and color rendering of the fabric**

| **Tested fabrics** | **Coordinate values L,a,b for fabric after washing** | | | **CRI score** | |
|---|---|---|---|---|---|
| | **L** | **a** | **b** | **before washing** | **after 1 washing** |
| Blue | 42.62±0.12 | -3.56±0.11 | -40.21±0.25 | 100 | 99.08 |
| Pink | 42.73±026 | 59.52±0.12 | 5.04±0.06 | 100 | 98.95 |

Preservation of color brightness is responsible for the correct perception of color shades of dense surfaces in the daytime and in the evening. Since some components in the composition of household detergents can destroy fabric dyes, for example, oxygen bleaches with bleach activators, the color rendering index CRI should be monitored before and after washing in order to preserve the original color and appearance of the product. The high content of surfactants increases the residual amount of substances on the fibers of the fabric, forms a large amount of foam and reduces the brightness of the color of the fabric, which significantly increases the wear of fabrics and color fading.

The composition of the universal concentrated stain remover for washing white and colored laundry with laccase in the composition retains the CRI index and, accordingly, the brightness of the color in the full spectrum of RGBW light. Laccase in the pre-treatment for dense surfaces not only effectively removes stains and soils based on melanoidins and/or phenolic organic acids, but also preserves the brightness of the color.

### Example 11 (not part of the present invention)

Checking the cleaning and whitening power of FtCl (Ft Cleaning) 300. In order to determine the whitening of the enamel on the Martindale tester (hereinafter referred to as the Tester), sets were studied - samples of solutions of toothpastes with and without laccase and toothbrushes according to the following indicator.

FtCl (Ft Cleaning) 300, characterizes the cleaning ability of the solution (paste) when performing 300 cleaning movements (mah) in a suspension of laccase. The indicator is used as an analogue of PCR (Pellicle Cleaning Ration) and allows you to quantify the cleansing and whitening effect of the solution (paste) with a single cleaning.

The minimum set of equipment for testing was represented by the following instruments:
1. Martindale tester (model for protective shield) produced by ATLAS;
2. KONICA MINOLTA CR-400 colorimeter or other colorimeter (spectrophotometer) with the ability to measure color in Lab coordinates and aperture mask size no more than d = 12 mm;
3. Analytical balance with an accuracy of 0.1 mg, the upper limit of weighing is not less than 300g.
4. Hydroxyapatite substrates (hereinafter referred to as HAP substrates) according to the size of the hole in the Martindale tester brush-board, with a whiteness value (L) of 90-94;
5. Coffee solution standardized for melanoidins and phenolic organic compounds.

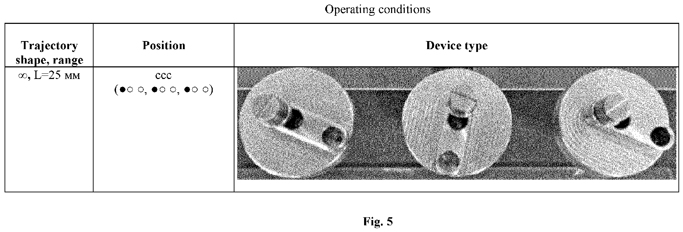

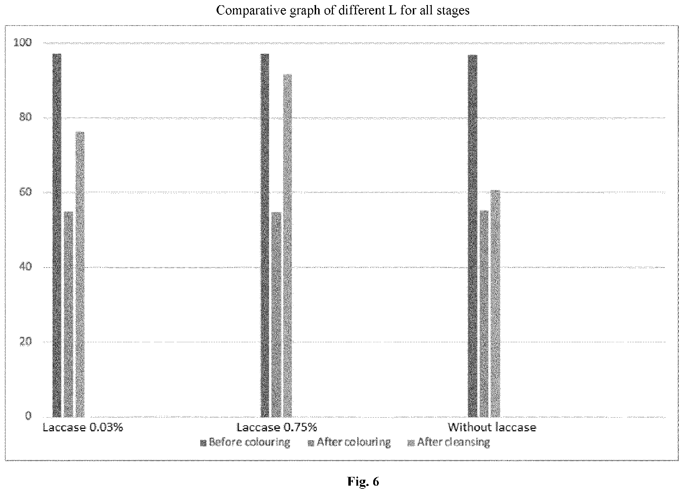

The operating conditions are illustrated in Figure 5.

Determination of FtCl300 for laccase solution:
1. trajectory: ∞ L=25 mm,
2. speed 150 mah/min,
3. impact - 300 mah,
4. brush load 325 g,
5. the minimum number of parallel determinations is one for each paste with a test brush,
6. test toothbrush: SPLAT PROFESSIONAL COMPLETE toothbrush medium hard
7. use one brush head for no more than 3-4 cycles, i.e. for 900-1200 mah, due to bristle wear; then change the brush head to a new one.

Conducting the test was based on preparation of hydroxyapatite disc substrates:
1. The quality of the substrates was assessed - all substrates with cracks, chips, traces of any geometric deformation were excluded;
2. Measured whiteness of the substrate surface (L₁);
3. The colorimeter is turned on and calibrated;
4. Checked instrument measurement system settings (this test requires Lab color coordinates);
5. Place the tablet on the measuring table, measure its whiteness three times;
6. Serial numbers are marked on the opposite side of the tablet;
7. Hydroxyapatite tablets are colored with a black coffee solution containing melanoidins and phenolic organic compounds, which give the coffee solution its characteristic color. To do this, the discs are immersed in 100 ml of a strong solution of black coffee standardized for melanoidins and phenolic organic compounds at 80-90°C for staining. After staining, the hydroxyapatite tablets are rinsed under running water and gently dried;
8. Measured the whiteness of the colored substrate (L₂).

Preparing the tester for testing:
1. Separate portions of the suspension of paste in water were prepared for each cell in a ratio of 1:2: 10 g of paste in 20 g of water;
2. Placed in the hole of the brush plate of the substrate with the painted side up, the suspensions are introduced. A tester for abrasion of substrates of colored hydroxyapatite discs was launched;
3. The substrates are dried with filter paper, then 5-10 min. dried in air, then - with an air dryer with an air temperature not higher than 40°C for 12-15 minutes, with periodic turning;
4. Recorded indicators of hydroxyapatite discs after cleaning - L₃.

FtCl300 values are calculated using the formula: $FtCl 300 = 100 * \left({L}_{3}-{L}_{2}\right) / \left({L}_{1}-{L}_{2}\right)$

Preparation of test solutions. Two toothpastes were prepared with the following formulations:
1. Toothpaste without active ingredients and with laccase 0.030%.
   Ingredients: Water, silica, preservative, thickener, laccase (0.030%)
2. Toothpaste without active ingredients and with laccase (0.750%)
   Ingredients: Water, silica, preservative, thickener, laccase (0.750%)
3. Toothpaste without active ingredients
   Ingredients: Water, silica, preservative, thickener.

For each composition, a solution was prepared from 10 g of paste and 20 g of purified water.

The level of lightness is estimated according to the CIE LAB scale. The Lab color model was created by the International Commission on Illumination (CIE). Color is not described in terms of elements reproduced by devices but using the three components of human color vision. In this model, any color is defined by lightness (L-Lightness) and two chromatic components: a channel is the colors from dark green through gray to magenta, the b channel is the colors from blue through gray to yellow. The a and b channels change from -128 to 127, and the L parameter from 0 to 100. The zero value of the color components at a brightness of 50 corresponds to gray in the RGB model (119,119,119). A brightness value of 100 produces white, while 0 produces black.

The level of purification is estimated by the formula FtCl300, where:
L₁ is the value of the whiteness of hydroxyapatite discs before staining;
L₂ is the whiteness value of hydroxyapatite discs after staining;
L₃ is whiteness value of hydroxyapatite discs after cleaning with toothpaste solutions.
$FtCl 300 = 100 * \left({L}_{3}-{L}_{2}\right) / \left({L}_{1}-{L}_{2}\right) ,$ where the main variable is L - Lightness, which at a value of 100 means white, so the higher the FtCl value, the better the cleaning of the surface of the hydroxyapatite discs.

### Results.

Thus, as a result of the study, it was found that the addition of laccase significantly improves the bleaching of hydroxyapatite discs dyed with coffee containing melanoidins and phenolic organic compounds, while the toothpaste without the addition of laccase showed practically no bleaching results (Table 16). The addition of laccase in toothpaste increased the teeth whitening by 1.5 times.

**Table 16. Evaluation of the L coordinate (whitening) for all stages**

| 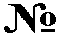 | | **Before colouring** | **Δ** | **After melanoidins (coffee) colouring** | **Δ** | **After cleansing** | **Δ** |
|---|---|---|---|---|---|---|---|
| | | L₁ | | L₂ | | L₃ | |
| 1 | Toothpaste with laccase 0.030 wt.% | 97.60 | 97.27 | 54.33 | 54.80 | 76.70 | 76.36 |
| 2 | | 97.45 | | 55.70 | | 76.83 | |
| 3 | | 96.80 | | 54.68 | | 76.50 | |
| 4 | | 97.21 | | 54.50 | | 75.40 | |
| 1 | Toothpaste with laccase 0.750 wt.% | 98.70 | 97.24 | 54.30 | 54.76 | 91.30 | 91.76 |
| 2 | | 96,50 | | 54.50 | | 92.60 | |
| 3 | | 97,56 | | 55.21 | | 91.78 | |
| 4 | | 96,21 | | 55.02 | | 91.34 | |
| 1 | Toothpaste without laccase | 97.80 | 96.80 | 56.10 | 55.20 | 61.03 | 60.67 |
| 2 | | 96.10 | | 54.30 | | 60.00 | |
| 3 | | 97.90 | | 55.20 | | 60.66 | |
| 4 | | 95.40 | | 55.20 | | 60.98 | |

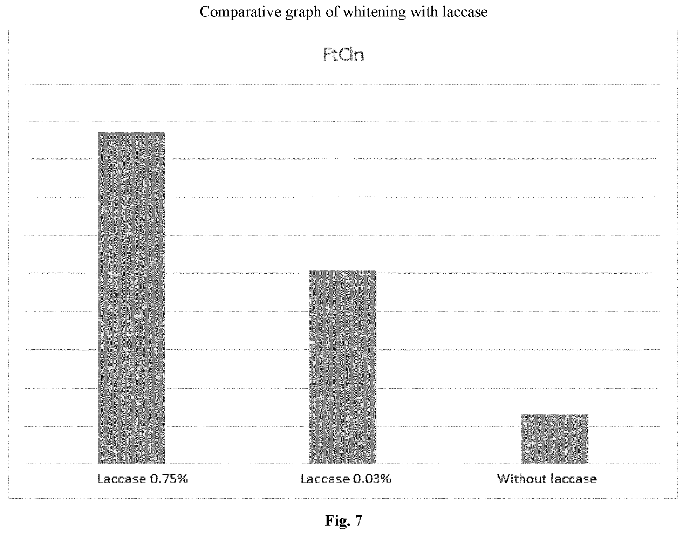

As a result of the research, the paste solutions showed themselves as follows (in descending order of the FtCl value): Laccase 0.750 wt.% > Laccase 0.030 wt.% > no Laccase. It can be visually compared at Fig. 7 and Table 17.

**Table 17. Evaluation of the FtCl for all stages**

| **Test specimen** | **Active components** | **FtCl score** | |
|---|---|---|---|
| | | **after cleaning** | **change, %** |
| Sample 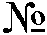 1 toothpaste | Without laccase | 13.14 | - |
| Sample 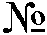 2 toothpaste | Laccase 0.030 wt.% | 50.76 | +286.3% |
| Sample 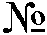 3 toothpaste | Laccase 0.750 wt.% | 87.08 | +562.7% |

The ability of laccase to decompose phenolic and non-phenolic compounds, such as melanoidins, significantly expands the area of potential application of the enzyme in oral care products. Simple and complex melanoidins are a group of heterogeneous substances that are widely distributed among food products: in coffee, cocoa beans, chocolate, bakery products, dark beer, wine, nuts and seeds, soy sauce, balsamic vinegar and other products that are dyes for tooth enamel. The use of laccase as a bioenzymatic whitening agent allows complex coffee stains to be removed from the teeth.

## Claims

1. Use of a composition comprising
a) a native laccase having the CAS number 80498-15-3, wherein said laccase is selected from *Aspergillus oryzae*;
and optionally,
b) a serine protease;
in the degradation of
- melanoidins, preferably food melanoidins,
and/or
- phenolic organic compounds, including monophenols, diphenols, methoxyphenols, and aminophenols.

2. The use of the composition of claim 1, wherein said serin protease is subtilisin, preferably subtilisin produced from Bacillus subtilis, further preferably subtilisin having the CAS number 9014-01-1.

3. The use of the composition of any of the preceding claims, wherein said composition is for use
- at a pH of 3 to 8, preferably 3.0 to 7.0, 3.5 to 6.5, or 7 to 8, and/or
- at a temperature range of +10 °C to +60 °C, preferably +20 °C to +60 °C.

4. The use of the composition of any of the preceding claims, wherein said melanoidin and/or phenolic organic compounds are comprised by the following to be treated:
- blood,
- sauce,
- grass,
- juice,
- jam,
- tea,
- cacao,
- coffee,
- wine,
- beer, in particular dark beer,
- food comprising nuts, seeds, cocoa beans,
- chocolate,
- soy sauce,
- balsamic vinegar,
- baking residues,
wherein said components are preferably contaminations on surfaces.

5. The use of the composition of any of the preceding claims, wherein said composition is for further use selected from at least one of the following:
a) as a laundry washing composition, preferably for the treatment of clothes, fabrics, and textiles, wherein said clothes, fabrics and textiles are further preferably selected from the group consisting of: cotton, synthetic, membrane, wool, silk, cashmere, merino, down, feather and mixtures thereof;
b) as a cleanser for the treatment of surfaces contaminated with melanoidin and/or phenolic compounds, including as a dishwashing cleanser, preferably as a dishwashing cleanser for automatic dishwashing machines;
c) as a lightener for lightening surfaces comprising melanoidins and/or phenolic compounds, wherein said washing composition or lightener is preferably for the treatment of surfaces having a density of at least 15.07 g/m² corresponding to fine silk;
d) as tooth lightener and/or tooth whitener, including said use as a toothpaste, wherein said tooth lightener and/or tooth whitener is for non-medical and/or aesthetic use, including its use for cleaning, lightening or whiting natural and/or non-natural tooth surfaces.

6. The use of the composition of any of the preceding claims, wherein said laccase comprising composition is
- in a gel, paste or liquid form, preferably in a water-glycerine, water-sorbitol, glycerine-sorbitol, water-propylene-glycol or water-maltitol solution form, or
- in a powdered or granulated form.

7. The use of the composition of any of the preceding claims, claims, wherein said composition comprises 0.03 to 1.00 wt%, preferably 0.10 to 0.75 wt%, further preferably 0.25 to 0.50 wt%, further preferably 0.50 wt% of said laccase in said composition.

8. The use of the composition of any of the preceding claims, wherein said composition is assembled as a single composition comprising all said components, including said laccase and, if present, said serine protease.

9. The use of the composition of any of the preceding claims, said composition not comprising at least one of the following components:
- 1,4-dioxane,
- phosphates,
- a laccase inhibitor,
- a chelating agent, wherein said chelating agent is preferably EDTA.

10. A method for cleaning a surface, wherein said surface comprises melanoidins, preferably food melanoidins, and/or phenolic organic compounds, said method comprising:
a) providing a gel, paste or liquid composition, wherein said composition
i) comprises laccase, wherein said laccase is a native laccase having the CAS number 80498-15-3, wherein said laccase is selected from *Aspergillus oryzae;* and
wherein said composition comprises 0.03 to 1.00 wt%, preferably 0.10 to 0.75 wt%, further preferably 0.25 to 0.50 wt%, further preferably 0.50 wt% of said laccase in said composition;
ii) optionally comprises a serine proteinase wherein said serin protease is subtilisin, preferably subtilisin produced from Bacillus subtilis, further preferably subtilisin having the CAS number 9014-01-1; and
iii) has a pH of 3 to 8, preferably 3.0 to 7.0, further preferably 3.5 to 6.5; or 7 to 8;
and
b) applying said composition to said surface, preferably in a temperature range of +10 °C to +60 °C, further preferably in a temperature range of +20 °C to +60 °C.

11. The method of claim 10, wherein said composition is selected from at least one of the following:
a) said composition is a laundry washing composition, preferably for the treatment of clothes, fabrics, and textiles, wherein said clothes, fabrics and textiles are further preferably selected from the group consisting of: cotton, synthetic, membrane, wool, silk, cashmere, merino, down, feather and mixtures thereof;
b) said composition is a cleanser for the treatment of surfaces contaminated with melanoidin and/or phenolic compounds, including natural and/or non-natural tooth surfaces or as a dishwashing cleanser, preferably as a dishwashing cleanser for automatic dishwashing machines;
c) said composition is a lightener for lightening surfaces comprising melanoidins and/or phenolic compounds.

12. The method of claim 10 or claim 11, wherein said composition is in a water-glycerine, water-sorbitol, glycerine-sorbitol, water-propylene-glycol or water-maltitol solution form.

## Patentansprüche

1. Verwendung einer Zusammensetzung, umfassend
a) eine native Laccase, welche die CAS-Nummer 80498-15-3 aufweist, wobei die Laccase aus *Aspergillus oryzae* ausgewählt ist;
und optional
b) einer Serinprotease;
beim Abbau von
- Melanoidinen, vorzugsweise Lebensmittel-Melanoidinen,
und/oder
- phenolischer organischer Verbindungen, einschließlich Monophenole, Diphenole, Methoxyphenole und Aminophenole.

2. Verwendung der Zusammensetzung nach Anspruch 1, wobei die Serinprotease Subtilisin ist, vorzugsweise Subtilisin, das des Weiteren aus *Bacillus subtilis* hergestellt wird, weiter bevorzugt Subtilisin mit der CAS-Nummer 9014-01-1.

3. Verwendung der Zusammensetzung nach einem der vorangegangenen Ansprüche, wobei die Zusammensetzung zur Verwendung
- bei einem pH-Wert von 3 bis 8, vorzugsweise 3,0 bis 7,0, 3,5 bis 6,5 oder 7 bis 8, und/oder
- in einem Temperaturbereich von +10 °C bis +60 °C, vorzugsweise von +20 °C bis +60 °C, vorgesehen ist.

4. Verwendung der Zusammensetzung nach einem der vorangegangenen Ansprüche, wobei das Melanoidin und/oder die phenolischen organischen Verbindungen umfasst sind von folgendem zu behandelnden:
- Blut,
- Soße,
- Gras,
- Saft,
- Marmelade,
- Tee,
- Kakao,
- Kaffee,
- Wein,
- Bier, insbesondere dunkles Bier,
- Lebensmittel umfassend Nüsse, Samen, Kakaobohnen,
- Schokolade,
- Sojasauce,
- Balsamico-Essig,
- Backrückstände,
wobei die genannten Komponenten vorzugsweise Verunreinigungen auf Oberflächen sind.

5. Verwendung der Zusammensetzung nach einem der vorangegangenen Ansprüche, wobei die Zusammensetzung zur des Weiteren Verwendung aus mindestens einem der folgenden Bereiche ausgewählt ist:
a) als Waschmittel, vorzugsweise zur Behandlung von Kleidung, Stoffen und Textilien, wobei die Kleidung, Stoffe und Textilien des Weiteren vorzugsweise aus der Gruppe ausgewählt sind, bestehend aus: Baumwolle, Synthetik, Membran, Wolle, Seide, Kaschmir, Merino, Daunen, Federn und Mischungen davon;
b) als Reinigungsmittel zur Behandlung von Oberflächen, die mit Melanoidinen und/oder phenolischen Verbindungen verunreinigt sind, einschließlich als Geschirrspülmittel, vorzugsweise als Geschirrspülmittel für Geschirrspülmaschinen;
c) als Aufheller zum Aufhellen von Oberflächen, die Melanoidine und/oder phenolische Verbindungen umfassen, wobei die Waschmittelzusammensetzung oder der Aufheller vorzugsweise zur Behandlung von Oberflächen mit einer Dichte von mindestens 15,07 g/m² bestimmt ist, was feiner Seide entspricht;
d) als Zahnaufheller und/oder Zahnweißmittel, einschließlich der Verwendung als Zahnpasta, wobei der Zahnaufheller und/oder das Zahnweißmittel für nichtmedizinische und/oder ästhetische Zwecke bestimmt ist, einschließlich seiner Verwendung zur Reinigung, Aufhellung oder zum Weißmachen natürlicher und/oder nicht-natürlicher Zahnoberflächen.

6. Verwendung der Zusammensetzung nach einem der vorangegangenen Ansprüche, wobei die Laccase umfassende Zusammensetzung
- in Gel-, Pasten- oder flüssiger Form vorliegt, vorzugsweise in Form einer Wasser-Glycerin-, Wasser-Sorbitol-, Glycerin-Sorbitol-, Wasser-Propylenglykol- oder Wasser-Maltitol-Lösung, oder
- in Pulver- oder Granulatform vorliegt.

7. Verwendung der Zusammensetzung nach einem der vorangegangenen Ansprüche, wobei die Zusammensetzung 0,03 bis 1,00 Gew.-%, vorzugsweise 0,10 bis 0,75 Gew.-%, weiter vorzugseise 0,25 bis 0,50 Gew.-%, weiter vorzugsweise 0,50 Gew.-% der Laccase in der Zusammensetzung umfasst.

8. Verwendung der Zusammensetzung nach einem der vorangegangenen Ansprüche, wobei die Zusammensetzung als eine einzige Zusammensetzung zusammengestellt ist, die alle genannten Komponenten umfasst, einschließlich der Laccase und, falls vorhanden, der Serinprotease.

9. Verwendung der Zusammensetzung nach einem der vorangegangenen Ansprüche, wobei die Zusammensetzung mindestens eine der folgenden Komponenten nicht umfasst:
- 1,4-Dioxan,
- Phosphate,
- einen Laccase-Inhibitor,
- eines Chelatbildners, wobei der Chelatbildner vorzugsweise EDTA ist.

10. Verfahren zur Reinigung einer Oberfläche, wobei die Oberfläche Melanoidine, vorzugsweise Lebensmittel-Melanoidine, und/oder phenolische organische Verbindungen umfasst, wobei das Verfahren umfasst:
a) Bereitstellen einer Gel-, Pasten- oder Flüssigzusammensetzung, wobei die Zusammensetzung
i) Laccase umfasst, wobei die Laccase eine native Laccase mit der CAS-Nummer 80498-15-3 ist, wobei die Laccase aus *Aspergillus oryzae* ausgewählt ist; und
wobei die Zusammensetzung 0,03 bis 1,00 Gew.-%, vorzugsweise 0,10 bis 0,75 Gew.- %, weiter vorzugsweise 0,25 bis 0,50 Gew.-%, weiter vorzugsweise 0,50 Gew.-% der Laccase in der Zusammensetzung umfasst;
ii) optional eine Serinprotease umfasst, wobei die Serinprotease Subtilisin ist, vorzugsweise von *Bacillus subtilis* hergestelltes Subtilisin, weiter vorzugsweise Subtilisin mit der CAS-Nummer 9014-01-1; und
iii) einen pH-Wert von 3 bis 8, weiter vorzugsweise von 3,0 bis 7,0, weiter vorzugsweise von 3,5 bis 6,5; oder von 7 bis 8 aufweist;
und
b) Aufbringen der Zusammensetzung auf die Oberfläche, vorzugsweise in einem Temperaturbereich von +10 °C bis +60 °C, weiter vorzugsweise in einem Temperaturbereich von +20 °C bis +60 °C.

11. Verfahren nach Anspruch 10, wobei die Zusammensetzung aus mindestens einer der folgenden ausgewählt ist:
a) die Zusammensetzung ist eine Waschmittelzusammensetzung, vorzugsweise zur Behandlung von Kleidung, Stoffen und Textilien, wobei die Kleidung, Stoffe und Textilien weiter vorzugsweise aus der Gruppe ausgewählt sind, bestehend aus: Baumwolle, Synthetik, Membran, Wolle, Seide, Kaschmir, Merino, Daunen, Federn und Mischungen davon;
b) die Zusammensetzung ist ein Reinigungsmittel zur Behandlung von mit Melanoidinen und/oder phenolischen Verbindungen verunreinigten Oberflächen, einschließlich natürlicher und/oder nicht-natürlicher Zahnoberflächen, oder als Geschirrspülmittel, vorzugsweise als Geschirrspülmittel für Geschirrspülmaschinen;
c) die Zusammensetzung ist ein Aufheller zum Aufhellen von Oberflächen, die Melanoidine und/oder phenolische Verbindungen umfassen.

12. Verfahren nach Anspruch 10 oder Anspruch 11, wobei die Zusammensetzung in Form einer Wasser-Glycerin-, Wasser-Sorbitol-, Glycerin-Sorbitol-, Wasser-Propylenglykol- oder Wasser-Maltitol-Lösung vorliegt.

## Revendications

1. Utilisation d'une composition comprenant
a) une laccase native ayant le numéro CAS 80498-15-3, où ladite laccase est choisie parmi Aspergillus oryzae ; et éventuellement,
b) une sérine protéase ;
dans la dégradation de
- mélanoïdines, de préférence des mélanoïdines alimentaires,
et/ou
- de composés organiques phénoliques, incluant les monophénols, les diphénols, les méthoxyphénols, les aminophénols.

2. Utilisation de la composition selon la revendication 1, dans laquelle ladite sérine protéase est une subtilisine, de préférence une subtilisine produite à partir de Bacillus subtilis, de préférence encore la subtilisine ayant le numéro CAS 9014-01-1.

3. Utilisation de la composition selon l'une quelconque des revendications précédentes, dans laquelle ladite composition est destinée à être utilisée
- à un pH de 3 à 8, de préférence 3,0 à 7,0, 3,5 à 6,5, ou 7 à 8, et/ou
- à une plage de température de +10 °C à +60 °C, de préférence de +20 °C à +60 °C.

4. Utilisation de la composition selon l'une quelconque des revendications précédentes, dans laquelle ladite mélanoïdine et/ou lesdits composés organiques phénoliques sont compris dans les produits suivants à traiter :
- sang,
- sauce,
- herbe,
- jus,
- confiture,
- thé,
- cacao,
- café,
- vin,
- bière, en particulier bière brune,
- produit alimentaire comprenant des noix, des graines, des fèves de cacao,
- chocolat,
- sauce soja,
- vinaigre balsamique,
- résidus de cuisson,
lesdits composants étant de préférence des contaminations sur des surfaces.

5. Utilisation de la composition selon l'une quelconque des revendications précédentes, ladite composition étant pour utilisation supplémentaire choisie parmi au moins l'un des suivants :
a) en tant que composition pour le lavage du linge, de préférence pour le traitement de vêtements, tissus et textiles, dans laquelle lesdits vêtements, tissus et textiles sont de préférence en outre choisis dans le groupe constitué par : coton, synthétique, membrane, laine, soie, cachemire, mérinos, duvet, plumes et leurs mélanges ;
b) comme nettoyant pour le traitement de surfaces contaminées par de la mélanoïdine et/ou des composés phénoliques, y compris comme nettoyant pour lave-vaisselle, de préférence comme nettoyant pour lave-vaisselle pour des lave-vaisselles automatiques ;
c) en tant qu'agent éclaircissant pour éclaircir des surfaces comprenant des mélanoïdines et/ou des composés phénoliques, ladite composition lavante ou ledit agent éclaircissant étant de préférence pour le traitement de surfaces présentant une masse volumique d'au moins 15,07 g/m² correspondant à de la soie fine ;
d) en tant qu'éclaircissant dentaire et/ou blanchissant dentaire, y compris ladite utilisation en tant que dentifrice, ledit éclaircissant dentaire et/ou blanchissant dentaire étant destiné à une utilisation non médicale et/ou esthétique, y compris son utilisation pour nettoyer, éclaircir ou blanchir des surfaces dentaires naturelles et/ou non naturelles.

6. Utilisation de la composition selon l'une quelconque des revendications précédentes, dans laquelle ladite composition comprenant une laccase est
- sous forme de gel, de pâte ou de liquide, de préférence sous forme de solution eau-glycérine, eau-sorbitol, glycérine-sorbitol, eau-propylène-glycol ou eau-maltitol, ou
- sous forme pulvérulente ou granulée.

7. Utilisation de la composition selon l'une quelconque des revendications précédentes, dans laquelle ladite composition comprend 0,03 à 1,00 % en poids, de préférence 0,10 à 0,75 % en poids, de préférence encore 0,25 à 0,50 % en poids, de préférence encore 0,50 % en poids de ladite laccase dans ladite composition.

8. Utilisation de la composition selon l'une quelconque des revendications précédentes, dans laquelle ladite composition est assemblée sous la forme d'une composition unique comprenant tous lesdits composants, y compris ladite laccase et, si elle est présente, ladite sérine protéase.

9. Utilisation de la composition de l'une quelconque des revendications précédentes, ladite composition ne comprenant pas au moins l'un des composants suivants :
- 1,4-dioxane,
- phosphates,
- un inhibiteur de laccase,
- un agent chélatant, où ledit agent chélatant est de préférence l'EDTA.

10. Procédé de nettoyage d'une surface, dans lequel ladite surface comprend des mélanoïdines, de préférence des mélanoïdines alimentaires, et/ou des composés organiques phénoliques, ledit procédé comprenant :
a) fourniture d'une composition de gel, de pâte ou de liquide, dans lequel ladite composition
i) comprend une laccase, ladite laccase étant une laccase native ayant le numéro CAS 80498-15-3, ladite laccase étant choisie parmi Aspergillus oryzae ; et dans lequel ladite composition comprend 0,03 à 1,00 % en poids, de préférence 0,10 à 0,75 % en poids, de préférence encore 0,25 à 0,50 % en poids, de préférence encore 0,50 % en poids de ladite laccase dans ladite composition ;
ii) éventuellement comprend une sérine protéinase, où ladite sérine protéase est une subtilisine, de préférence une subtilisine produite à partir de Bacillus subtilis, de préférence encore la subtilisine ayant le numéro CAS 9014-01-1 ; et
iii) présente un pH de 3 à 8, de préférence de 3,0 à 7,0, de préférence encore de 3,5 à 6,5 ; ou 7 à 8 ;
et
b) application de ladite composition sur ladite surface, préférablement dans une plage de température de +10 °C à +60 °C, encore préférablement dans une plage de température de +20 °C à +60 °C.

11. Procédé selon la revendication 10, dans lequel ladite composition est choisie parmi au moins l'une des suivantes :
a) ladite composition est une composition pour le lavage du linge, de préférence pour le traitement de vêtements, tissus et textiles, lesdits vêtements, tissus et textiles étant de préférence choisis dans le groupe constitué par : coton, synthétique, membrane, laine, soie, cachemire, mérinos, duvet, plumes et leurs mélanges ;
b) ladite composition est un agent nettoyant pour le traitement de surfaces contaminées par de la mélanoïdine et/ou des composés phénoliques, y compris les surfaces naturelles et/ou non naturelles des dents ou en tant que nettoyant pour lave-vaisselle, de préférence comme nettoyant pour lave-vaisselle pour des lave-vaisselles automatiques ;
c) ladite composition est un agent éclaircissant pour l'éclaircissement de surfaces comprenant des mélanoïdines et/ou des composés phénoliques.

12. Procédé selon la revendication 10 ou la revendication 11, dans lequel ladite composition est sous forme de solution eau-glycérine, eau-sorbitol, glycérine-sorbitol, eau-propylène-glycol ou eau-maltitol.
